# EUROPEAN PATENT SPECIFICATION

(11) **EP 3 290 526 B1**
(45) Date of publication and mention of the grant of the patent: **19.11.2025**
(21) Application number: 15890778.2
(22) Date of filing: 11.12.2015
(51) Int. Cl.: A01H 6/82, C12Q 1/68, C12N 15/00, C12Q 1/6895

(54) **MARKER FOR LEAF MOLD RESISTANCE IN TOMATO PLANT, LEAF MOLD-RESISTANT TOMATO PLANT, METHOD FOR PRODUCING LEAF MOLD-RESISTANT TOMATO PLANT, AND METHOD FOR SCREENING FOR LEAF MOLD-RESISTANT TOMATO PLANT**
MARKER FÜR SAMTFLECKENRESISTENZ BEI TOMATENPFLANZEN, SAMTFLECKENRESISTENTE TOMATENPFLANZE, VERFAHREN ZUR HERSTELLUNG EINER SAMTFLECKENRESISTENTEN TOMATENPFLANZE UND SCREENING-VERFAHREN FÜR SAMTFLECKENRESISTENTE TOMATENPFLANZE
MARQUEUR POUR LA RÉSISTANCE À LA MOISISSURE DES FEUILLES DANS UNE PLANTE DE TOMATE, PLANTE DE TOMATE RÉSISTANTE À LA MOISISSURE DES FEUILLES, PROCÉDÉ DE PRODUCTION DE PLANTE DE TOMATE RÉSISTANTE À LA MOISISSURE DES FEUILLES, ET PROCÉDÉ DE CRIBLAGE DE PLANTE DE TOMATE RÉSISTANTE À LA MOISISSURE DES FEUILLES

(30) Priority: 30.04.2015 JP 2015093528
(43) Date of publication of application: 07.03.2018
(73) Proprietor: Takii & Company Limited, Kyoto 600-8686 (JP)
(72) Inventor: YOSHIDA Takuji, Kyoto-shi Kyoto 600-8686 (JP); ARIMOTO Ryohei, Kyoto-shi Kyoto 600-8686 (JP); ENDO Makoto, Kyoto-shi Kyoto 600-8686 (JP); AOIKE Hitomi, Kyoto-shi Kyoto 600-8686 (JP)
(74) Representative: J A Kemp LLP
(86) International application number: PCT/JP2015/084750
(87) International publication number: WO 2016/174795

(56) References cited:
- US-A1- 2010 043 085
- BENJAMIN STICH ET AL: "Population structure and linkage disequilibrium in diploid and tetraploid potato revealed by genome-wide high-density genotyping using the SolCAP SNP array", PLANT BREEDING., vol. 132, no. 6, 3 October 2013 (2013-10-03), DE, pages 718 - 724, XP055517800, ISSN: 0179-9541, DOI: 10.1111/pbr.12102
- HAANSTRA ET AL: "Mapping strategy for resistance genes against Cladosporium fulvum on the short arm of Chromosome 1 of tomato: Cf- ECP5 near the Hcr9 Milky Way cluster", THEOR APPL GENET., vol. 1, no. 4, 10 September 2000 (2000-09-10), pages 661 - 668, XP055326498, DOI: 10.1007/s001220051528
- TINGTING ZHAO ET AL: "Mapping and candidate gene screening of tomato Cladosporium fulvum-resistant gene Cf-19, based on high-throughput sequencing technology", BMC PLANT BIOLOGY, vol. 16, no. 1, 25 February 2016 (2016-02-25), XP055517442, DOI: 10.1186/s12870-016-0737-0
- BARAN N. ET AL: "Studies on the minimal lengths required for DNA primers to be extended by the Tetrahymena telomerase: implications for primer positioning by the enzyme", NUCLEIC ACIDS RESEARCH, vol. 30, no. 24, 15 December 2002 (2002-12-15), GB, pages 5570 - 5578, XP093236446, ISSN: 1362-4962, Retrieved from the Internet <URL:https://pmc.ncbi.nlm.nih.gov/articles/PMC140050/pdf/gkf676.pdf> DOI: 10.1093/nar/gkf676
- HAANSTRA, J.P .W. ET AL.: "Mapping strategy for resistance genes against Cladosporium fulvum on the short arm of Chromosome 1 of tomato: Cf- ECP5 near the Hcr9 Milky Way cluster", THEOR APPL GENET., vol. 1, no. 4, 10 September 2000 (2000-09-10), pages 661 - 668, XP055326498
- SOUMPOUROU,E. ET AL.: "The Solanum pimpinellifolium Cf-ECP1 and Cf-ECP4 genes for resistance to Cladosporium fulvum are located at the Milky Way locus on the short arm of chromosome 1", THEOR APPL GENET., vol. 115, no. 8, 11 November 2007 (2007-11-11), pages 1127 - 1136, XP019558609
- DATABASE GenBank 20 November 1998 (1998-11-20), DIXON,M.S. ET AL.: "DEFINITION: Lycopersicon pimpinellifolium Hcr2- 2A (Hcr2-2A) gene , complete cds.", XP055326503, Database accession no. AF053996

## Description

### Technical Field

The present disclosure relates to a leaf mold resistance marker for tomato plants, a leaf mold resistant tomato plant, a method for producing a leaf mold resistant tomato plant, and a screening method for leaf mold resistant tomato plants.

### Background Art

In cultivation of tomato plants, a disease caused by the leaf mold fungus is a serious problem worldwide. It has been reported that, in a plant body infected with the leaf mold fungus, most of mature leaves die and the plant body cannot bear fruits (Non-Patent Document 1). Accordingly, prevention and extermination of the leaf mold fungus are performed using agricultural chemicals. This, however, requires a large amount of labor and cost.

Also, attempts have been made to breed tomato cultivars resistant to the leaf mold fungus utilizing leaf mold resistance genes. However, appearance of new types of leaf mold fungus capable of infecting tomato plants including these resistance genes gives rise to a new problem (Non-Patent Documents 2 and 3).

### Citation List

### Non-Patent Document(s)

[Non-Patent Document 1] "LEAF MOLD OF GREENHOUSE TOMATOES", University of Illinois report on PLANT DISEASE, Integrated Pest Management at the University of Illinois, August 1989
[Non-Patent Document 2] Junichiro Enya et.al., "The first occurrence of leaf mold of tomato caused by races 4.9 and 4.9.11 of Passalora fulva (syn. Fulvia fulva) in Japan", J. Gen. Plant. Pathol., Springer, 2009, vol.75, pp. 76-79
[Non-Patent Document 3] "Occurrence of tomato leaf mold races 2.9, 4.9, 2.5.9, and 4.5.9", Byogaichu Hassei Yosatsu Tokusyu-hou (Special Report on Forecast of Pest Occurrence) No. 1, 2014, Tochigi Prefectural Sustainable Agriculture Extension Center, July 24, 2014

### Brief Summary of the Invention

The invention provides a use of a leaf mold resistance marker for identifying at least one of a leaf mold resistance locus from chromosome 6 of a tomato plant and a leaf mold resistance locus from chromosome 1 of a tomato plant,
which marker for identifying the leaf mold resistance locus from chromosome 6 of a tomato plant is at least one SNP marker selected from the group consisting of: (a) solcap_snp_sl_25252, (b) solcap_snp_sl_35221, and (c) SG_70, wherein:
   (a) is a polynucleotide consisting of a base sequence of SEQ ID NO: 3;
   (b) is a polynucleotide consisting of a base sequence of SEQ ID NO: 4; and
   (c) is a polynucleotide consisting of a base sequence of SEQ ID NO: 5, and
which marker for identifying the leaf mold resistance locus from chromosome 1 of a tomato plant is at least one SNP marker selected from the group consisting of: (d) solcap_snp_sl_60160 and (e) solcap_snp_sl_8658, wherein:
   (d) is a polynucleotide consisting of a base sequence of SEQ ID NO: 1; and
   (e) is a polynucleotide consisting of a base sequence of SEQ ID NO: 2.

The invention further provides a leaf mold resistant tomato plant comprising: at least one of a leaf mold resistance locus on chromosome 1 characterized by a leaf mold resistance marker as defined above and a leaf mold resistance locus on chromosome 6 characterized by a leaf mold resistance marker as defined above, wherein the leaf mold resistance locus is present in a tomato plant identified by Accession No. FERM BP-22282.

Yet further provided by the invention is a screening method for a leaf mold resistant tomato plant, the screening method to select a parent for producing a leaf mold resistant tomato plant, the screening method comprising the step of, selecting a leaf mold resistant tomato plant comprising at least one of a leaf mold resistance locus on chromosome 1 and a leaf mold resistance locus on chromosome 6 from one or more tomato plants to be examined,
wherein the leaf mold resistance locus on chromosome 1 is identified using at least one SNP marker selected from the group consisting of (d) solcap_snp_sl_60160 and (e) solcap_snp_sl_8658,wherein:
   (d) is a polynucleotide consisting of a base sequence of SEQ ID NO: 1; and
   (e) is a polynucleotide consisting of a base sequence of SEQ ID NO: 2,
and the leaf mold resistance locus on chromosome 6 is identified using at least one SNP marker selected from the group consisting of (a) solcap_snp_sl_25252, (b) solcap_snp_sl_35221, and (c) SG_70, wherein:
   (a) is a polynucleotide consisting of a base sequence of SEQ ID NO: 3;
   (b) is a polynucleotide consisting of a base sequence of SEQ ID NO: 4; and
   (c) is a polynucleotide consisting of a base sequence of SEQ ID NO: 5.

Further aspects and preferred embodiments are defined in the dependent claims. Any aspects, embodiments and examples of the present disclosure which do not fall under the scope of the appended claims do not form part of the invention and are merely provided for illustrative purposes.

### Brief Summary of Technical Teachings

### Problem to be Solved by the Disclosure

With the foregoing in mind, it is an object of the present disclosure to provide a novel leaf mold resistance marker for tomato plants, exhibiting dominantly inherited leaf mold resistance; a leaf mold resistant tomato plant including at least one dominant leaf mold resistance locus; a method for producing a leaf mold resistant tomato plant using the same; and a screening method for leaf mold resistant tomato plants.

### Means for Solving Problem

Disclosed herein is a leaf mold resistance marker for a tomato plant, including: at least one of a leaf mold resistance locus on chromosome 1 and a leaf mold resistance locus on chromosome 6, wherein the leaf mold resistance locus on chromosome 1 is identified by at least one SNP marker selected from the group consisting of solcap_snp_sl_60160, solcap_snp_sl_8658,and solcap_snp_sl_8658', and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70'.

Further disclosed herein is a leaf mold resistant tomato plant including: at least one of a leaf mold resistance locus on chromosome 1 and a leaf mold resistance locus on chromosome 6, wherein the leaf mold resistance locus on chromosome 1 is identified by at least one SNP marker selected from the group consisting of solcap_snp_sl_60160, solcap_snp_sl_8658,and solcap_snp_sl_8658', and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70'.

Further disclosed herein is a method for producing a leaf mold resistant tomato plant, including the following steps (a) and (b):
(a) crossing the leaf mold resistant tomato plant according to the present disclosure with another tomato plant; and
(b) selecting a leaf mold resistant tomato plant from one or more tomato plants obtained in the step (a) or progeny lines thereof.

Further disclosed herein is a screening method for a leaf mold resistant tomato plant, including the step of: as a parent for producing a leaf mold resistant tomato plant by crossing, selecting a leaf mold resistant tomato plant including, as a leaf mold resistance marker for a tomato plant, at least one of a leaf mold resistance locus on chromosome 1 and a leaf mold resistance locus on chromosome 6 from one or more tomato plants to be examined, wherein the leaf mold resistance locus on chromosome 1 is identified by at least one SNP marker selected from the group consisting of solcap_snp_sl_60160, solcap_snp_sl_8658,and solcap_snp_sl_8658', and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70'.

### Effects of the Disclosure

The invention is defined in the appended claims. Any aspects, embodiments and examples of the present disclosure which do not fall under the scope of the appended claims do not form part of the invention and are merely provided for illustrative purposes.
The inventors conducted diligent studies, and discovered two leaf mold resistance loci as leaf mold resistance markers for tomato plants, exhibiting dominantly inherited leaf mold resistance. Then, the inventors found out that the first novel leaf mold resistance locus (also referred to as " first resistance locus " hereinafter) is located on chromosome 1, and is identified (also referred to as "specified" hereinafter) by at least one SNP marker selected from the group consisting of solcap_snp_sl_60160, solcap_snp_sl_8658, and solcap_snp_sl_8658'. The inventors also found out that the second novel leaf mold resistance locus (also referred to as "second resistance locus" hereinafter) is located on chromosome 6, and is identified by at least one SNP marker selected from the group consisting of solcap_snp_sl_25252, solcap_snp_sl_25252', solcap_snp_sl_35221, SG_70, and SG_70'. Tomato plant including the leaf mold resistance marker(s) exhibits dominantly inherited leaf mold resistance. Thus, the leaf mold resistance marker for tomato plants according to the present disclosure enables easy screening for leaf mold resistant tomato plants, for example. Also, the leaf mold resistant tomato plant according to the present disclosure includes, for example, at least one of the first resistance locus and the second resistance locus, and thus can exhibit leaf mold resistance, for example. Further, since the leaf mold resistant tomato plant of the present disclosure includes, for example, at least one dominant resistance locus, it is possible to obtain progenies exhibiting leaf mold resistance inherited dominantly by crossing the leaf mold resistant tomato plant of the present disclosure with other tomato plants. Furthermore, a tomato plant including the leaf mold resistance marker is resistant to races of leaf mold fungus capable of infecting tomato plants including the leaf mold resistance genes disclosed in the prior art documents, for example. Thus, the present disclosure can eliminate the necessity of prevention and extermination using agricultural chemicals as performed conventionally, so that the problem of labor and cost for spraying the agricultural chemicals can be avoided, for example.

### Brief Description of Drawings

[FIG. 1] FIG. 1 is a schematic view showing relative locations of single nucleotide polymorphism (SNP) markers on chromosome 1.
[FIG. 2] FIG. 2 is a schematic view showing relative locations of SNP markers on chromosome 6.

### Mode for Carrying out the Disclosure

### 1. Leaf mold resistance marker for tomato plants

The leaf mold resistance marker for a tomato plant (also referred to as "resistance marker" hereinafter) disclosed herein is, as described above, a leaf mold resistance marker for a tomato plant, including at least one of a leaf mold resistance locus on chromosome 1 and a leaf mold resistance locus on chromosome 6, wherein the leaf mold resistance locus on chromosome 1 is identified by at least one SNP marker selected from the group consisting of solcap_snp_sl_60160, solcap_snp_sl_8658,and solcap_snp_sl_8658', and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70'. The resistance marker disclosed herein is characterized in that: it includes at least one of a leaf mold resistance locus on chromosome 1 and a leaf mold resistance locus on chromosome 6; the leaf mold resistance locus on chromosome 1 is identified by at least one SNP marker selected from the group consisting of solcap_snp_sl_60160, solcap_snp_sl_8658, and solcap_snp_sl_8658'; and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70', and other configurations or conditions are not particularly limited.

In the present disclosure, "tomato plants" are plants classified in *Section Lycopersicon* in the subgenus *Solmum sensu stricto* of the genus *Solanum.* Specific examples thereof include *S. lycopersicum, S. peruvianum, S. arcanum Peralta, S. chilense, S. corneliomulleri, S. huaylasense Peralta, S. cheesmaniae* (L. Riley) Fosberg, *S. chmielewskii, S. galapagense S.* C. Darwin & Peralta, *S. habrochaites, S. neorickii, S. pennelli,* and *S. pimpinellifolium.* Among them, *S. lycopersicum* is preferable because it can be crossed easily.

In the present disclosure, the leaf mold fungus is a filamentous fungus, for example. The filamentous fungus is, for example, *Fulvia fulva.* The *Fulvia fulva* is also referred to as *Passalora fulva* or *Cladosporium fulvum,* for example.

The term "leaf mold resistance" as used herein also may be referred to as "leaf mold tolerance", for example. The resistance means the ability to inhibit or suppress the occurrence and progression of damage due to the infection with the pathogen of the leaf mold, for example. Specifically, the resistance may mean any of the following, for example: to prevent the damage from occurring; to stop the progression of the damage that has occurred already; and to suppress (also referred to as "inhibit") the progression of the damage that has occurred already.

The leaf mold resistance marker disclosed herein includes at least one of the leaf mold resistance locus on chromosome 1 and the leaf mold resistance locus on chromosome 6, as described above. When a tomato plant including the leaf mold resistance marker includes the first resistance locus, the tomato plant having the leaf mold resistance may include the first resistance locus on, instead of chromosome 1, any chromosome other than chromosome 1, for example. That is, the tomato plant including the first resistance locus may include the first resistance locus on any of chromosome 2, chromosome 3, chromosome 4, chromosome 5, chromosome 6, chromosome 7, chromosome 8, chromosome 9, chromosome 10, chromosome 11, and chromosome 12. When a tomato plant including the leaf mold resistance marker includes the second resistance locus, the tomato plant having the leaf mold resistance may include the second resistance locus on, instead of chromosome 6, any chromosome other than chromosome 6, for example. That is, the tomato plant including the second resistance locus may include the second resistance locus on any of chromosome 1, chromosome 2, chromosome 3, chromosome 4, chromosome 5, chromosome 7, chromosome 8, chromosome 9, chromosome 10, chromosome 11, and chromosome 12. When a tomato plant having the leaf mold resistance includes the first resistance locus and the second resistance locus, the tomato plant including the leaf mold resistance loci may include the first resistance locus and the second resistance locus on the same chromosome or on different chromosomes.

The leaf mold resistance locus refers to a quantitative trait locus or a gene region that confers the leaf mold resistance. In general, the quantitative trait locus (QTL) refers to a chromosome region that is involved in the expression of a quantitative trait. The QTL can be specified using a molecular marker that indicates a specific locus on a chromosome. The technique for specifying the QTL using the molecular marker is well known in the art.

In the present disclosure, a molecular marker used for specifying the leaf mold resistance locus is not particularly limited. Examples of the molecular marker include SNP markers, amplified fragment length polymorphism (AFLP) markers, restriction fragment length polymorphism (RFLP) markers, microsatellite markers, sequence-characterized amplified region (SCAR) markers, and cleaved amplified polymorphic sequence (CAPS) markers.

In the present disclosure, one SNP marker may be used, or two or more SNP markers may be used in combination, for example.

The resistance marker disclosed herein may include only the first resistance locus or only the second resistance locus. When a tomato plant including the resistance marker includes both the first resistance locus and the second resistance locus, the tomato plant exhibits further improved leaf mold resistance, for example. Specifically, a tomato plant in which each of the first resistance locus and the second resistance locus is in the heterozygous form with resistant and susceptible alleles exhibits higher leaf mold resistance than a tomato plant in which the first resistance locus is in a homozygous form with resistant alleles (referred to as "resistance-type homozygous form" hereinafter) and exhibits equivalent leaf mold resistance to a tomato plant in which the second resistance locus is in the resistance-type homozygous form, for example. Also, with this configuration, for example, by crossing the tomato plant including the first resistance locus and the second resistance locus with a tomato plant having any other resistance, it is possible to produce a tomato plant having high leaf mold resistance and the other resistance easily. Therefore, the resistance marker disclosed herein preferably includes the leaf mold resistance locus on chromosome 1 and the leaf mold resistance locus on chromosome 6. The respective resistance loci will be described specifically below.

### (1) First resistance locus

In the present disclosure, the first resistance locus is identified by (1-1) the SNP marker(s), as described above. Alternatively, the first resistance locus may be specified by: (1-2) a base sequence including the SNP marker(s); (1-3) a base sequence in a region between sites of two SNP markers; or any combination of (1-1) to (1-3). When the first resistance locus is specified by any combination of (1-1) to (1-3), the combination is not particularly limited, and examples thereof include the combinations shown below. Although the first resistance locus is identified by (1-1), the identification of the first resistance locus is not limited thereto. For example, instead of (1-1), the first resistance locus may be identified by (1-2) or (1-3), or the combination of (1-2) and (1-3).
Combination of (1-1) and (1-2)
Combination of (1-1) and (1-3)
Combination of (1-1), (1-2), and (1-3)

### (1-1) Identification by SNP marker(s)

The first resistance locus is specified by the SNP marker(s), as shown in (1-1) above. The SNP marker is not particularly limited, and examples thereof include solcap_snp_sl_60160, solcap_snp_sl_8658, and solcap_snp_sl_8658'. SNP markers indicated as "solcap_snp_sl_[specific number]" excluding solcap_snp_sl_8658 are apparent to those skilled in the art in view of the common general technical knowledge as of the filing date of the present application. Information on the SNP markers is available on the web site of the SOL Genomics Network (http://solgenomics.net/). As to the analysis of these SNPs, the following Reference Documents 1 to 3 can be referred to, for example (the same applies hereinafter). The solcap_snp_sl_8658' is a SNP marker newly identified by the inventors, and those skilled in the art can identify the chromosomal location of this SNP marker on the basis of the base sequences including the SNP markers to be described below.
Reference Document 1: Hamilton JP, Sim SC, Stoffel K, Van Deynze A, Buell CR, et al. (2012) "Single Nucleotide Polymorphism Discovery in Cultivated Tomato via Sequencing by Synthesis.", The Plant Genome 5.
Reference Document 2: Sim S-C, Durstewitz G, Plieske J, Wieseke R, Ganal MW, et al., (2012) "Development of a Large SNP Genotyping Array and Generation of High-Density.", Genetic Maps in Tomato. PLoS ONE 7 (7)
Reference Document 3: Blanca J, Canizares J, Cordero L, Pascual L, Diez MJ, et al., (2012) "Variation Revealed by SNP Genotyping and Morphology Provides Insight into the Origin of the Tomato.", PLoS ONE 7 (10)

Solcap_snp_sl_60160 (also referred to as "SNP (a)" hereinafter) is a polymorphism such that the underlined base in brackets in SEQ ID NO: 1 is T, for example. That is, for example, a tomato plant is resistant to leaf mold when the underlined base is T, and is susceptible to leaf mold when the underlined base is other than T (e.g., C). The base sequence of SEQ ID NO: 1 can be obtained from a tomato plant deposited under Accession No. FERM BP-22282 to be described below, for example.

SEQ ID NO: 1
5'-
TGATGGAGGAAACATTACATTCTAATATTTTCGCAGCAAACATCTACAC[T]
GTTTGATGCTTTTAATGTATCAGCCTGTTATTCAGAA-3'

SNP (a) can be identified on the basis of known information in a database of the above-described web site etc., for example. The base sequence of SEQ ID NO: 6 is identical to the base sequence of Heinz (Cultivar Name: Heinz 1706) except for SNP (a), for example, and the underlined base in brackets is a polymorphism corresponding to SNP (a). That is, for example, a tomato plant is resistant to leaf mold when the underlined base is T, and is susceptible to leaf mold when the underlined base is other than T (e.g., C). Thus, the location of SNP (a) can be identified on the basis of information on the known base sequence of Heinz, for example.

SEQ ID NO: 6
5'-
GTGATGGAGGAAACATTACATTCTAATATTTTCGCAGCAAACATCTACAC[
T]GTTTGATGCTTTTAATGTATCAGCCTGTTATTCAGAAGCTGTCTTCGTT
T-3'

Solcap_snp_sl_8658 (also referred to as "SNP (b)" hereinafter) is a polymorphism such that the underlined base in brackets in SEQ ID NO: 2 is T, for example. That is, for example, a tomato plant is resistant to leaf mold when the underlined base is T, and is susceptible to leaf mold when the underlined base is other than T (e.g., C). The base sequence of SEQ ID NO: 2 can be obtained from the tomato plant deposited under Accession No. FERM BP-22282 to be described below, for example.
SEQ ID NO: 2 5' -TTTTGGTTAAGTGTGGTGTCGCIGACCAATTTATGCACAT[T]TTGATTAATCTACTGAGCACTTGCTACCTACTA-3'

SNP (b) can be identified on the basis of known information in the database of the above-described web site etc., for example. The base sequence of SEQ ID NO: 7 is identical to the base sequence of Heinz except for SNP (b) and solcap_snp_sl_8658 to be described below, for example, and the underlined base in brackets is a polymorphism corresponding to SNP (b). That is, for example, a tomato plant is resistant to leaf mold when the underlined base is T, and is susceptible to leaf mold when the underlined base is other than T (e.g., C). Thus, the location of SNP (b) can be identified on the basis of information on the known base sequence of Heinz, for example.

SEQ ID NO : 7
5' -TACTCAAACTTTTTTGGTTAAGTGTGGTGTCGCGACCAATTTATGCACAT[T]TTGATTAATCTACTGAGCACTTGCT
ACCTACTACCTATATATGTATTGGG-3'

Solcap_snp_sl_8658' (also referred to as "SNP (b')" hereinafter) is a polymorphism such that the boxed base in SEQ ID NO: 2 is C, for example. That is, for example, a tomato plant is resistant to leaf mold when the boxed base is C, and is susceptible to leaf mold when the boxed base is other than C (e.g., T).

SNP (b') can be identified on the basis of known information in the database of the above-described web site etc., for example. The base sequence of SEQ ID NO: 7 is identical to the base sequence of Heinz except for SNP (b) and SNP (b'), for example, and the boxed base is a polymorphism corresponding to SNP (b'). That is, for example, a tomato plant is resistant to leaf mold when the boxed base is C, and is susceptible to leaf mold when the boxed base is other than C (e.g., T). Thus, the location of SNP (b') can be identified on the basis of information on the known base sequence of Heinz, for example.

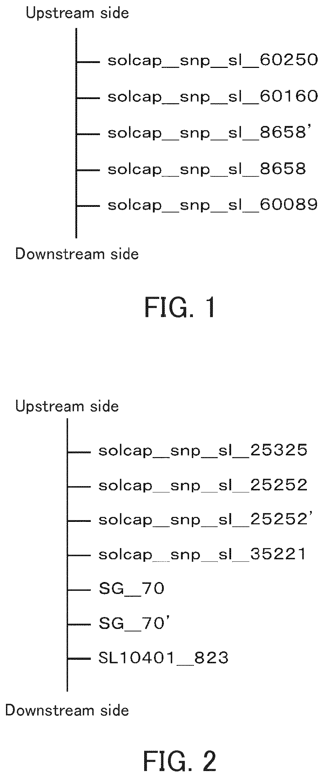

The locations of the SNP markers on the chromosome are not particularly limited. For example, as shown in FIG. 1, on chromosome 1 of a tomato plant, the SNP markers are located in the following order from the upstream side (the solcap_snp_sl_60250 side) toward the downstream side (the solcap_snp_sl_60089 side): solcap_snp_sl_60250, solcap_snp_sl_60160, solcap_snp_sl_8658', solcap_snp_sl_8658,and solcap_snp_sl_60089. Note here that solcap_snp_sl_60250 and solcap_snp_sl_60089 are used to indicate the locations of solcap_snp_sl_60160, solcap_snp_sl_8658', and solcap_snp_sl_8658 on chromosome 1 of the tomato plant. Solcap_snp_sl_60250 and solcap_snp_sl_60089 will be described below.

The number of the SNP markers present in the first resistance locus is not particularly limited. For example, the first resistance locus may include any one, two, or three of the SNP markers. The relevance of these three types of polymorphisms (SNP markers) with the leaf mold resistance has not been reported heretofore. They are novel polymorphisms discovered first by the inventors as being involved in the leaf mold resistance.

The combination of the SNP markers is not particularly limited, and examples thereof include the following combinations.
Combinations of two SNP markers
   SNP (a) and SNP (b)
   SNP (a) and SNP (b')
   SNP (b) and SNP (b')
Combination of three SNP markers
   SNP (a), SNP (b), and SNP (b')
Among the above combinations, the following combinations are preferable, for example, because they show higher correlation with the leaf mold resistance:
   SNP (a) and SNP (b)
   SNP (b) and SNP (b')
   SNP (a), SNP (b), and SNP (b')

### (1-2) Identification by base sequence including SNP marker(s)

The first resistance locus may be specified by a base sequence including the SNP marker(s), as shown in (1-2) above, for example. The first resistance locus may consist of the base sequence or may include the base sequence, for example.

The base sequence including the SNP marker(s) is not particularly limited, and examples thereof include the following polynucleotides (a) and (b). The polynucleotide (a) corresponds to a base sequence including SNP (a) as the SNP marker, and the polynucleotide (b) corresponds to a base sequence including at least one of SNP (b) and SNP (b') as the SNP marker(s).

The polynucleotide (a) is a base sequence including SNP (a), i.e., solcap_snp_sl_60160, and is, for example, the following polynucleotide (a1), (a2), or (a3). The polynucleotides (a2) and (a3) are polynucleotides each having a function equivalent to that of the polynucleotide (a1) regarding the leaf mold resistance in the first resistance locus.
(a1) a polynucleotide consisting of the base sequence of SEQ ID NO: 1
(a2) a polynucleotide consisting of a base sequence obtained by deletion, substitution, insertion, and/or addition of one or more bases excluding the 50th base (T) in the base sequence of the polynucleotide (a1)
(a3) a polynucleotide consisting of a base sequence having at least 80% sequence identity to the base sequence of the polynucleotide (a1) with the 50th base (T) in the base sequence of the polynucleotide (a1) being conserved

In the polynucleotide (a1), the underlined 50th base (T) in brackets in SEQ ID NO: 1 is a base corresponding to the polymorphism of solcap_snp_sl_60160. The polynucleotide (a1) can be obtained from the tomato plant deposited under Accession No. FERM BP-22282 to be described below, for example.

In the polynucleotide (a2), the number of the "one or more" bases is, for example, 1 to 18, 1 to 13, 1 to 10, 1 to 9, 1, 2, 3, or 4. In the present disclosure, the numerical range regarding the number of bases discloses all the positive integers falling within that range, for example. That is, for example, the description "one to five bases" discloses all of "one, two, three, four, and five bases" (the same applies hereinafter).

In the polynucleotide (a3), the "sequence identity" is, for example, at least 80%, at least 85%, at least 89%, at least 90%, at least 95%, at least 96%, at least 97%, at least 98%, or at least 99%. The "sequence identity" can be determined by aligning two base sequences (the same applies hereinafter).

The polynucleotide (b) is a base sequence including at least one of SNP (b) and SNP (b'), i.e., at least one of solcap_snp_sl_8658 and solcap_snp_sl_8658'. When the polynucleotide (b) is a base sequence including SNP (b) as the SNP marker, the polynucleotide (b) is, for example, the following polynucleotide (b1), (b2), or (b3). The polynucleotides (b2) and (b3) are polynucleotides each having a function equivalent to that of the polynucleotide (b1) regarding the leaf mold resistance in the first resistance locus.
(b1) a polynucleotide consisting of the base sequence of SEQ ID NO: 2
(b2) a polynucleotide consisting of a base sequence obtained by deletion, substitution, insertion, and/or addition of one or more bases excluding the 40th base (T) in the base sequence of the polynucleotide (b1)
(b3) a polynucleotide consisting of a base sequence having at least 80% sequence identity to the base sequence of the polynucleotide (b1) with the 40th base (T) in the base sequence of the polynucleotide (b1) being conserved

In the polynucleotide (b1), the underlined 40th base (T) in brackets in SEQ ID NO: 2 is a base corresponding to the polymorphism of solcap_snp_sl_8658. The polynucleotide (b1) can be obtained from the tomato plant deposited under Accession No. FERM BP-22282 to be described below, for example.

In the polynucleotide (b2), the number of the "one or more" bases is, for example, 1 to 15, 1 to 12, 1 to 9, 1 to 8, 1, 2, 3, or 4.

In the polynucleotide (b3), the "sequence identity" is, for example, at least 80%, at least 85%, at least 89%, at least 90%, at least 95%, at least 96%, at least 97%, at least 98%, or at least 99%.

When the polynucleotide (b) is a base sequence including SNP (b') as the SNP marker, the polynucleotide (b) is, for example, the following polynucleotide (b1'), (b2'), or (b3'). The polynucleotides (b2') and (b3') are polynucleotides each having a function equivalent to that of the polynucleotide (b1') regarding the leaf mold resistance in the first resistance locus.
(b1') a polynucleotide consisting of the base sequence of SEQ ID NO: 2
(b2') a polynucleotide consisting of a base sequence obtained by deletion, substitution, insertion, and/or addition of one or more bases excluding the 22nd base (C) in the base sequence of the polynucleotide (b1')
(b3') a polynucleotide consisting of a base sequence having at least 80% sequence identity to the base sequence of the polynucleotide (b1') with the 22nd base (C) in the base sequence of the polynucleotide (b1') being conserved

In the polynucleotide (b1'), the boxed 22nd base (C) in SEQ ID NO: 2 is a base corresponding to the polymorphism of solcap_snp_sl_8658'. The polynucleotide (b1') can be obtained from the tomato plant deposited under Accession No. FERM BP-22282 to be described below, for example.

The above description regarding the number of the "one or more" bases in the polynucleotide (b2) also applies to the number of the "one or more" bases in the polynucleotide (b2'), for example.

The above description regarding the "sequence identity" in the polynucleotide (b3) also applies to the "sequence identity" in the polynucleotide (b3'), for example.

When the polynucleotide (b) is a base sequence including SNP (b) and SNP (b') as the SNP markers, the polynucleotide (b) is, for example, the following polynucleotide (b1"), (b2"), or (b3"). The polynucleotides (b2") and (b3") are polynucleotides each having a function equivalent to that of the polynucleotide (b1") regarding the leaf mold resistance in the first resistance locus.
(b1") a polynucleotide consisting of the base sequence of SEQ ID NO: 2
(b2") a polynucleotide consisting of a base sequence obtained by deletion, substitution, insertion, and/or addition of one or more bases excluding the 22nd base (C) and the 40th base (T) in the base sequence of the polynucleotide (b1")
(b3") a polynucleotide consisting of a base sequence having at least 80% sequence identity to the base sequence of the polynucleotide (b1") with the 22nd base (C) and the 40th base (T) in the base sequence of the polynucleotide (b1") being conserved

In the polynucleotide (b1"), the underlined 40th base (T) in brackets and the boxed 22nd base (C) in SEQ ID NO: 2 are bases corresponding to the polymorphisms of solcap_snp_sl_8658 and solcap_snp_sl_8658', respectively. The polynucleotide (b1") can be obtained from the tomato plant deposited under Accession No. FERM BP-22282 to be described below, for example.

The above description regarding the number of the "one or more" bases in the polynucleotide (b2) also applies to the number of the "one or more" bases in the polynucleotide (b2"), for example.

The above description regarding the "sequence identity" in the polynucleotide (b3) also applies to the "sequence identity" in the polynucleotide (b3"), for example.

The number of the base sequences including the SNP marker(s) in the first resistance locus is not particularly limited. For example, the first resistance locus may include either one of the polynucleotides (a) and (b), or both of these two polynucleotides (a) and (b).

The combination of the base sequences including the SNP marker(s) is not particularly limited, and examples thereof include the following combinations.

### Combination of the polynucleotide (a) and the polynucleotide (b)

### (1-3) Identification by base sequence in region between sites of two SNP markers

The first resistance locus may be specified by the base sequence in a region between sites of two SNP markers, as shown in (1-3) above, for example. The base sequence in a region between sites of the two SNP markers is not particularly limited, and examples thereof include a base sequence in a region between sites of two SNP markers selected from the group consisting of solcap_snp_sl_60250, solcap_snp_sl_60160, solcap_snp_sl_8658,solcap_snp_sl_8658', and solcap_snp_sl_60089 on the chromosome and a base sequence in a region between sites of two SNP markers selected from the group consisting of solcap_snp_sl_60250, solcap_snp_sl_60160, solcap_snp_sl_8658, and solcap_snp_sl_60089.

Solcap_snp_sl_60250 is a polymorphism at the underlined base in brackets in SEQ ID NO: 11, for example. In the tomato plant deposited under Accession No. FERM BP-22282 to be described below, solcap_snp_sl_60250 is a polymorphism such that the underlined base in brackets is G. However, solcap_snp_sl_60250 may be a polymorphism such that the underlined base in brackets is other than G, i.e., A, T, or C, for example. The base sequence of SEQ ID NO: 11 can be obtained from the tomato plant deposited under Accession No. FERM BP-22282 to be described below, for example.

SEQ ID NO: 11
5'-
GATGAACTATGGAGAAATACTGTAGTGAGTAAATATTTGAGAATAAGCA[G
]ATAGATGTAAGATAAAATGAATACATGACAAGA-3'

Solcap_snp_sl_60250 can be identified on the basis of known information in the database of the above-described web site etc., for example. The base sequence of SEQ ID NO: 12 is identical to the base sequence of Heinz except for solcap_snp_sl_60250, for example, and the underlined base in brackets is a polymorphism corresponding to solcap_snp_sl_60250. Thus, the location of solcap_snp_sl_60250 can be identified on the basis of information on the known base sequence of Heinz, for example.

SEQ ID NO: 12
5'-
GATGAACTATGGAGAAATACTGTAGTGAGTAAATATTTGAGAATAAGCA[G
]ATAGATGTAAGATAAAATGAATACATGACAAGA-3'

Solcap_snp_sl_60089 is a polymorphism at the underlined base in brackets in SEQ ID NO: 13, for example. In the tomato plant deposited under Accession No. FERM BP-22282 to be described below, solcap_snp_sl_60089 is a polymorphism such that the underlined base in brackets is C. However, solcap_snp_sl_60089 may be a polymorphism such that the underlined base in brackets is other than C, i.e., A, T, or G, for example. The base sequence of SEQ ID NO: 13 can be obtained from the tomato plant deposited under Accession No. FERM BP-22282 to be described below, for example.

SEQ ID NO: 13
5'-
TTTCGTGTTTCGTATTTAGAACTTCTTAATGAAAAT[C]CTGGCTCAGCAG
CTGGTAGTTAGTAGAGTGATTTGTGGGTGTATAT-3'

Solcap_snp_sl_60089 can be identified on the basis of known information in the database of the above-described web site etc., for example. The base sequence of SEQ ID NO: 14 is identical to the base sequence of Heinz except for solcap_snp_sl_60089, for example, and the underlined base in brackets is a polymorphism corresponding to solcap_snp_sl_60089. Thus, the location of solcap_snp_sl_60089 can be identified on the basis of information on the known base sequence of Heinz, for example.

SEQ ID NO: 14
5'-

TTTCGTGTTTCGTATTTAGAACTTCTTAATGAAAAT[C]CTGGCTCCGCCG
CTGGTAGTTAGTAGAGTGATTTGTGGGTGTATAT-3'

The upstream-side end and the downstream-side end of the region can be identified by, for example, the sites of the two SNP markers, as described above. The region is not limited as long as it extends between the sites of the two SNP markers, for example. The region may or may not include both or one of the sites of the two SNP markers, for example. When the region includes the sites of the SNP markers, the upstream-side end and the downstream-side end of the region are the sites of the SNP markers. The bases at the upstream-side end and the downstream-side end may each be the above-described underlined base in the base sequence or may be a base other than the underlined base, for example.

The two SNP markers specifying the region are not particularly limited, and examples thereof include the following combinations.
Combination of solcap_snp_sl_60250 and solcap_snp_sl_60160
Combination of solcap_snp_sl_60250 and solcap_snp_sl_8658
Combination of solcap_snp_sl_60250 and solcap_snp_sl_8658
Combination of solcap_snp_sl_60250 and solcap_snp_sl_60089
Combination of solcap_snp_sl_60160 and solcap_snp_sl_8658
Combination of solcap_snp_sl_60160 and solcap_snp_sl_8658
Combination of solcap_snp_sl_60160 and solcap_snp_sl_60089
Combination of solcap_snp_sl_8658' and solcap_snp_sl_60089
Combination of solcap_snp_sl_8658' and solcap_snp_sl_8658
Combination of solcap_snp_sl_8658 and solcap_snp_sl_60089

In the case where the first resistance locus is specified by the base sequence in a region between sites of the two SNP markers, it is preferable that the first resistance locus further includes, in the base sequence in the region, the SNP marker(s) located in the region. Specifically, it is preferable that the first resistance locus includes, in the base sequence in the region, at least one SNP marker selected from the group consisting of solcap_snp_sl_60160, solcap_snp_sl_8658,and solcap_snp_sl_8658', for example.

The SNP marker(s) located in the region may be, for example, one or both of the sites of the two SNP markers specifying the region on the chromosome, or may be the SNP marker(s) located between the sites of the two SNP markers specifying the region. The former also is referred to as a SNP marker at the end of the region, and the latter also is referred to as a SNP marker inside the region. The SNP markers located in the region may be both the SNP markers at the ends of the region and the SNP marker inside the region, for example.

The SNP marker(s) inside the region may be, for example, a SNP marker(s) located between the SNP marker on the upstream side and the SNP marker on the downstream side specifying the region, and can be determined as appropriate on the basis of the locations of the SNP markers shown in FIG. 1, for example. The number of the SNP markers between the sites of the two SNP markers may be one or more, for example. Specifically, for example, the SNP markers may be all the SNP markers located between the sites of the SNP markers specifying the region.

The combination of the base sequence in a region between sites of the two SNP markers and the SNP marker(s) in the base sequence in the region is not particularly limited, and examples thereof include the following condition (i):

### Condition (i)

The first resistance locus includes a base sequence in a region between sites of solcap_snp_sl_60250 and solcap_snp_sl_60089 on the chromosome, and
the first resistance locus includes, in the base sequence in the region, at least one SNP marker selected from the group consisting of solcap_snp_sl_60160, solcap_snp_sl_8658, and solcap_snp_sl_8658'.

In the condition (i), the number of the SNP markers inside the region is not particularly limited. For example, the region may include any one of solcap_snp_sl_60160, solcap_snp_sl_8658,and solcap_snp_sl_8658', or two or more (i.e., two or three) of them. When the region includes two or more of the SNP markers, the combination of the SNP markers is not particularly limited, and examples thereof include the following combinations.
Combinations of two SNP markers:
   SNP (a) and SNP (b)
   SNP (a) and SNP (b')
   SNP (b) and SNP (b')
Combination of three SNP markers:
   SNP (a), SNP (b), and SNP (b')

### (2) Second resistance locus

In the present disclosure, the second resistance locus is specified by (2-1) the SNP marker(s), as described above. Alternatively, the second resistance locus may be specified by: (2-2) a base sequence including the SNP marker(s); (2-3) a base sequence in a region between sites of two SNP markers; or any combination of (2-1) to (2-3). When the second resistance locus is specified by any combination of (2-1) to (2-3), the combination is not particularly limited, and examples thereof include the combinations shown below. Although the second resistance locus is identified by (2-1), the identification of the second resistance locus is not limited thereto. For example, instead of (2-1), the second resistance locus may be identified by (2-2) or (2-3), or the combination of (2-2) and (2-3).
Combination of (2-1) and (2-2)
Combination of (2-1) and (2-3)
Combination of (2-1), (2-2), and (2-3)

### (2-1) Identification by SNP marker(s)

The second resistance locus may be specified by the SNP marker(s), as shown in (2-1) above, for example. The SNP marker is not particularly limited, and examples thereof include solcap_snp_sl_25252, solcap_snp_sl_25252', solcap_snp_sl_35221, SG_70, and SG_70'. Information on the SNP markers indicated as "solcap_snp_sl_[specific number]" excluding solcap_snp_sl_25252' is available on the above-described web site. The solcap_snp_sl_25252', SG_70, and SG_70' are SNP markers newly identified by the inventors, and those skilled in the art can identify the chromosomal locations of these SNP markers on the basis of the base sequences including the SNP markers to be described below.

Solcap_snp_sl 25252 (also referred to as "SNP (c)" hereinafter) is a polymorphism such that the underlined base in brackets in SEQ ID NO: 3 is G, for example. That is, for example, a tomato plant is resistant to leaf mold when the underlined base is G, and is susceptible to leaf mold when the underlined base is other than G (e.g., A). The base sequence of SEQ ID NO: 3 can be obtained from the tomato plant deposited under Accession No. FERM BP-22282 to be described below, for example.

SEQ ID NO : 3
5' -TGGGTGTCGTAAAAGTCTCATAATTGAGATCTC[G]CGTTTGATATTTGCCATTAAAATCGCGTTGCTAACT-3'

SNP (c) can be identified on the basis of known information in the database of the above-described web site etc., for example. The base sequence of SEQ ID NO: 8 is identical to the base sequence of Heinz except for SNP (c) and solcap_snp_sl_25252' to be described below, for example, and the underlined base in brackets is a polymorphism corresponding to SNP (c). That is, for example, a tomato plant is resistant to leaf mold when the underlined base is G, and is susceptible to leaf mold when the underlined base is other than G (e.g., A). Thus, the location of SNP (c) can be identified on the basis of information on the known base sequence of Heinz, for example.

SEQ ID NO : 8
5' -TCAAGTTTGACAATGTGTGGGTGTCGTAAAAGTCTCATAATTGAGATCTC[G]CGTTTGATATTTGCCATTAAAATCG
CGTTGCTAACTCTTGTTTTGTTAAT-3'

Solcap_snp_sl_25252' (also referred to as "SNP (c')" hereinafter) is a polymorphism such that the boxed base in SEQ ID NO: 3 is T, for example. That is, for example, a tomato plant is resistant to leaf mold when the boxed base is T, and is susceptible to leaf mold when the boxed base is other than T (e.g., C).

SNP (c') can be identified on the basis of known information in the database of the above-described web site etc., for example. The base sequence of SEQ ID NO: 8 is identical to the base sequence of Heinz except for SNP (c) and SNP (c'), for example, and the boxed base is a polymorphism corresponding to SNP (c'). That is, for example, a tomato plant is resistant to leaf mold when the boxed base is T, and is susceptible to leaf mold when the boxed base is other than T (e.g., C). Thus, the location of SNP (c') can be identified on the basis of information on the known base sequence of Heinz, for example.

Solcap_snp_sl_35221 (also referred to as "SNP (d)" hereinafter) is a polymorphism such that the underlined base in brackets in SEQ ID NO: 4 is A, for example. That is, for example, a tomato plant is resistant to leaf mold when the underlined base is A, and is susceptible to leaf mold when the underlined base is other than A (e.g., G). The base sequence of SEQ ID NO: 4 can be obtained from the tomato plant deposited under Accession No. FERM BP-22282 to be described below, for example.

SEQ ID NO: 4
5'-
TCTAATACCATCTGCAAGTTTCTGAGCCTC[A]TCTGACTTCAGCGGACAT
CTACTTAAG-3'

SNP (d) can be identified on the basis of known information in the database of the above-described web site etc., for example. The base sequence of SEQ ID NO: 9 is identical to the base sequence of Heinz except for SNP (d), for example, and the underlined base in brackets is a polymorphism corresponding to SNP (d). That is, for example, a tomato plant is resistant to leaf mold when the underlined base is A, and is susceptible to leaf mold when the underlined base is other than A (e.g., G). Thus, the location of SNP (d) can be identified on the basis of information on the known base sequence of Heinz, for example.

SEQ ID NO: 9
5'-
CTCGAAATTGATAAGTTTTCTCTAATACCATCTGCAAGTTTCTGAGCCTC[
A]TCTGACTTCAGCGGACATCTACTTAAGGTCTGTAAAACAATCAACTTTA
T-3'

SG_70 (also referred to as "SNP (e)" hereinafter) is a polymorphism such that the underlined base in brackets in SEQ ID NO: 5 is A, for example. That is, for example, a tomato plant is resistant to leaf mold when the underlined base is A, and is susceptible to leaf mold when the underlined base is other than A (e.g., G). The base sequence of SEQ ID NO: 5 can be obtained from the tomato plant deposited under Accession No. FERM BP-22282 to be described below, for example.

SEQ ID NO : 5
5' -ATAGTGAGTCAAACAATTGTACATTACATATCAATCACT[A]TGTTATCACAACTTCCAACTGATTACTAGCTTAAGA
CATC-3'

SNP (e) can be identified on the basis of known information in the database of the above-described web site etc., for example. The base sequence of SEQ ID NO: 10 is identical to the base sequence of Heinz except for SNP (e) and SG_70' to be described below, for example, and the underlined base in brackets is a polymorphism corresponding to SNP (e). That is, for example, a tomato plant is resistant to leaf mold when the underlined base is A, and is susceptible to leaf mold when the underlined base is other than A (e.g., G). Thus, the location of SNP (e) can be identified on the basis of information on the known base sequence of Heinz, for example.

SEQ ID NO : 10
5' -GAACCAAATCCATTACGCCAATGCGTTCTTATTCTTGGCATTTGAAAATCTGAAAAATGAGATAGTGAGTCAAACAAC
TGTACATTACATATCAATCACT[A]TGTTATCACAACTTCCAACTGATTACTAGCTTAAGACATCAGCTAAGGCTTAATCTT
CAGCTTCTTGAAGTTGTTGGAAAGAAAAATATGCTTCGTATCG-3'

SG_70' (also referred to as "SNP (e')" hereinafter) is a polymorphism such that the boxed base in SEQ ID NO: 5 is G, for example. That is, for example, a tomato plant is resistant to leaf mold when the boxed base is G, and is susceptible to leaf mold when the boxed base is other than G (e.g., A).

SNP (e') can be identified on the basis of known information in the database of the above-described web site etc., for example. The base sequence of SEQ ID NO: 10 is identical to the base sequence of Heinz except for SNP (e) and SNP (e'), for example, and the boxed base is a polymorphism corresponding to SNP (e'). That is, for example, a tomato plant is resistant to leaf mold when the boxed base is G, and is susceptible to leaf mold when the boxed base is other than G (e.g., A). Thus, the location of SNP (e') can be identified on the basis of information on the known base sequence of Heinz, for example.

The locations of the SNP markers on the chromosome are not particularly limited. For example, as shown in FIG. 2, on chromosome 6 of a tomato plant, the SNP markers are located in the following order from the upstream side (the solcap_snp_sl_25325 side) toward the downstream side (the SL10401_823 side): solcap_snp_sl_25325, solcap_snp_sl_25252, solcap_snp_sl_25252', solcap_snp_sl_35221, SG_70, SG_70', and SL10401_823. Note here that solcap_snp_sl_25325 and SL10401_823 are used to indicate the locations of solcap_snp_sl_25252, solcap_snp_sl_25252', solcap_snp_sl_35221, SG_70, and SG_70' on chromosome 6 of the tomato plant. solcap_snp_sl_25325 and SL10401_823 will be described below.

The number of the SNP markers present in the second resistance locus is not particularly limited. For example, the second resistance locus may include any one of the SNP markers or two or more of the SNP marker (i.e., any two, three, or four of the SNP markers or all the five SNP markers). The relevance of these five types of polymorphisms (SNP markers) with the leaf mold resistance has not been reported heretofore. They are novel polymorphisms discovered first by the inventors as being involved in the leaf mold resistance.

The combination of the SNP markers is not particularly limited, and examples thereof include the following combinations.
Combinations of two SNP markers
   SNP (c) and SNP (c')
   SNP (c) and SNP (d)
   SNP (c) and SNP (e)
   SNP (c) and SNP (e')
   SNP (c') and SNP (d)
   SNP (c') and SNP (e)
   SNP (c') and SNP (e')
   SNP (d) and SNP (e)
   SNP (d) and SNP (e')
   SNP (e) and SNP (e')
Combinations of three SNP markers
   SNP (c), SNP (c'), and SNP (d)
   SNP (c), SNP (c'), and SNP (e)
   SNP (c), SNP (c'), and SNP (e')
   SNP (c), SNP (d), and SNP (e)
   SNP (c), SNP (d), and SNP (e')
   SNP (c), SNP (e), and SNP (e')
   SNP (c'), SNP (d), and SNP (e)
   SNP (c'), SNP (d), and SNP (e')
   SNP (c'), SNP (e), and SNP (e')
   SNP (d), SNP (e), and SNP (e')
Combinations of four SNP markers
   SNP (c), SNP (c'), SNP (d), and SNP (e)
   SNP (c), SNP (c'), SNP (d), and SNP (e')
   SNP (c), SNP (c'), SNP (e), and SNP (e')
   SNP (c), SNP (d), SNP (e), and SNP (e')
   SNP (c'), SNP (d), SNP (e), and SNP (e')
Combination of five SNP markers
   SNP (c), SNP (c'), SNP (d), SNP (e), and SNP (e')
Among the above combinations, the following combinations are preferable, for example, because they show higher correlation with the leaf mold resistance.
Combination of SNP (c) and SNP (c')
Combination of SNP (e) and SNP (e')
Combination of SNP (c), SNP (d), and SNP (e)
Combination of SNP (c), SNP (c'), SNP (d), SNP (e), and SNP (e')

### (2-2) Identification by base sequence including SNP marker(s)

The second resistance locus may be specified by a base sequence including the SNP marker(s), as shown in (2-2) above, for example. The second resistance locus may consist of the base sequence or may include the base sequence, for example.

The base sequence including the SNP marker(s) is not particularly limited, and examples thereof include the following polynucleotides (c), (d), and (e). The polynucleotide (c) corresponds to a base sequence including at least one of SNP (c) and SNP (c') as the SNP marker(s). The polynucleotide (d) corresponds to a base sequence including SNP (d) as the SNP marker. The polynucleotide (e) corresponds to a base sequence including at least one of SNP (e) and SNP (e') as the SNP marker(s).

The polynucleotide (c) is a base sequence including at least one of SNP (c) and SNP (c'), i.e., at least one of solcap_snp_sl_25252 and solcap_snp_sl_25252'. When the polynucleotide (c) is a base sequence including SNP (c) as the SNP marker, the polynucleotide (c) is, for example, the following polynucleotide (c1), (c2), or (c3). The polynucleotides (c2) and (c3) are polynucleotides each having a function equivalent to that of the polynucleotide (c1) regarding the leaf mold resistance in the second resistance locus.
(c1) a polynucleotide consisting of the base sequence of SEQ ID NO: 3
(c2) a polynucleotide consisting of a base sequence obtained by deletion, substitution, insertion, and/or addition of one or more bases excluding the 34th base (G) in the base sequence of the polynucleotide (c1)
(c3) a polynucleotide consisting of a base sequence having at least 80% sequence identity to the base sequence of the polynucleotide (c1) with the 34th base (G) in the base sequence of the polynucleotide (c1) being conserved

In the polynucleotide (c1), the underlined 34th base (G) in brackets in SEQ ID NO: 3 is a base corresponding to the polymorphism of solcap_snp_sl_25252. The polynucleotide (c1) can be obtained from the tomato plant deposited under Accession No. FERM BP-22282 to be described below, for example.

In the polynucleotide (c2), the number of the "one or more" bases is, for example, 1 to 14, 1 to 11, 1 to 8, 1 to 4, 1, 2, or 3.

In the polynucleotide (c3), the "sequence identity" is, for example, at least 80%, at least 85%, at least 89%, at least 90%, at least 95%, at least 96%, at least 97%, at least 98%, or at least 99%.

When the polynucleotide (c) is a base sequence including SNP (c') as the SNP marker, the polynucleotide (c) is, for example, the following polynucleotide (c1'), (c2'), or (c3'). The polynucleotides (c2') and the (c3') are polynucleotides each having a function equivalent to that of the polynucleotide (c1') regarding the leaf mold resistance in the second resistance locus.
(c1') a polynucleotide consisting of the base sequence of SEQ ID NO: 3
(c2') a polynucleotide consisting of a base sequence obtained by deletion, substitution, insertion, and/or addition of one or more bases excluding the 46th base (T) in the base sequence of the polynucleotide (c1')
(c3') a polynucleotide consisting of a base sequence having at least 80% sequence identity to the base sequence of the polynucleotide (c1') with the 46th base (T) in the base sequence of the polynucleotide (c1') being conserved

In the polynucleotide (c1'), the boxed 46th base (T) in SEQ ID NO: 3 is a base corresponding to the polymorphism of solcap_snp_sl_25252'. The polynucleotide (c1') can be obtained from the tomato plant deposited under Accession No. FERM BP-22282 to be described below, for example.

The above description regarding the number of the "one or more" bases in the polynucleotide (c2) also applies to the number of the "one or more" bases in the polynucleotide (c2'), for example.

The above description regarding the "sequence identity" in the polynucleotide (c3) also applies to the "sequence identity" in the polynucleotide (c3'), for example.

When the polynucleotide (c) is a base sequence including SNP (c) and SNP (c') as the SNP markers, the polynucleotide (c) is, for example, the following polynucleotide (c1"), (c2"), or (c3"). The polynucleotides (c2") and (c3") are polynucleotides each having a function equivalent to that of the polynucleotide (c1") regarding the leaf mold resistance in the second resistance locus.
(c1") a polynucleotide consisting of the base sequence of SEQ ID NO: 3
(c2") a polynucleotide consisting of a base sequence obtained by deletion, substitution, insertion, and/or addition of one or more bases excluding the 34th base (G) and the 46th base (T) in the base sequence of the polynucleotide (c1")
(c3") a polynucleotide consisting of a base sequence having at least 80% sequence identity to the base sequence of the polynucleotide (c1") with the 34th base (G) and the 46th base (T) in the base sequence of the polynucleotide (c1") being conserved

In the polynucleotide (c1"), the underlined 34th base (G) in brackets and the boxed 46th base (T) in SEQ ID NO: 3 are bases corresponding to the polymorphisms of solcap_snp_sl_25252 and solcap_snp_sl_25252', respectively. The polynucleotide (c1") can be obtained from the tomato plant deposited under Accession No. FERM BP-22282 to be described below, for example.

The above description regarding the number of the "one or more" bases in the polynucleotide (c2) also applies to the number of the "one or more" bases in the polynucleotide (c2"), for example.

The above description regarding the "sequence identity" in the polynucleotide (c3) also applies to the "sequence identity" in the polynucleotide (c3"), for example.

The polynucleotide (d) is a base sequence including SNP (d), i.e., solcap_snp_sl_35221, and is, for example, the following polynucleotide (d1), (d2), or (d3). The polynucleotides (d2) and (d3) are polynucleotides each having a function equivalent to that of the polynucleotide (d1) regarding the leaf mold resistance in the second resistance locus.
(d1) a polynucleotide consisting of the base sequence of SEQ ID NO: 4
(d2) a polynucleotide consisting of a base sequence obtained by deletion, substitution, insertion, and/or addition of one or more bases excluding the 31st base (A) in the base sequence of the polynucleotide (d1)
(d3) a polynucleotide consisting of a base sequence having at least 80% sequence identity to the base sequence of the polynucleotide (d1) with the 31st base (A) in the base sequence of the polynucleotide (d1) being conserved

In the polynucleotide (d1), the underlined 31st base (A) in brackets in SEQ ID NO: 4 is a base corresponding to the polymorphism of solcap_snp_sl_35221. The polynucleotide (d1) can be obtained from the tomato plant deposited under Accession No. FERM BP-22282 to be described below, for example.

In the polynucleotide (d2), the number of the "one or more" bases is, for example, 1 to 12, 1 to 9, 1 to 8, 1 to 4, 1, 2, or 3.

In the polynucleotide (d3), the "sequence identity" is, for example, at least 80%, at least 85%, at least 89%, at least 90%, at least 95%, at least 96%, at least 97%, at least 98%, or at least 99%.

The polynucleotide (e) is a base sequence including at least one of SNP (e) and SNP (e'), i.e., at least one of SG_70 and SG_70'. When the polynucleotide (e) is a base sequence including SNP (e) as the SNP marker, the polynucleotide (e) is, for example, the following polynucleotide (e1), (e2), or (e3). The polynucleotides (e2) and (e3) are polynucleotides each having a function equivalent to that of the polynucleotide (e1) regarding the leaf mold resistance in the second resistance locus.
(e1) a polynucleotide consisting of the base sequence of SEQ ID NO: 5
(e2) a polynucleotide consisting of a base sequence obtained by deletion, substitution, insertion, and/or addition of one or more bases excluding the 40th base (A) in the base sequence of the polynucleotide (e1)
(e3) a polynucleotide consisting of a base sequence having at least 80% sequence identity to the base sequence of the polynucleotide (e1) with the 40th base (A) in the base sequence of the polynucleotide (e1) being conserved

In the polynucleotide (e1), the underlined 40th base (A) in brackets in SEQ ID NO: 5 is a base corresponding to the polymorphism of the SG_70. The polynucleotide (e1) can be obtained from the tomato plant deposited under Accession No. FERM BP-22282 to be described below, for example.

In the polynucleotide (e2), the number of the "one or more" bases is, for example, 1 to 16, 1 to 12, 1 to 9, 1 to 8, 1, 2, 3, or 4.

In the polynucleotide (e3), the "sequence identity" is, for example, at least 80%, at least 85%, at least 89%, at least 90%, at least 95%, at least 96%, at least 97%, at least 98%, or at least 99%.

When the polynucleotide (e) is a base sequence including SNP (e') as the SNP marker, the polynucleotide (e) is, for example, the following polynucleotide (e1'), (e2'), or (e3'). The polynucleotides (e2') and (e3') are polynucleotides each having a function equivalent to that of the polynucleotide (e1') regarding the leaf mold resistance in the second resistance locus.
(e1') a polynucleotide consisting of the base sequence of SEQ ID NO: 5
(e2') a polynucleotide consisting of a base sequence obtained by deletion, substitution, insertion, and/or addition of one or more bases excluding the 75th base (G) in the base sequence of the polynucleotide (e1')
(e3') a polynucleotide consisting of a base sequence having at least 80% sequence identity to the base sequence of the polynucleotide (e1') with the 75th base (G) in the base sequence of the polynucleotide (e1') being conserved

In the polynucleotide (e1'), the boxed 75th base (G) in SEQ ID NO: 5 is a base corresponding to the polymorphism of the SG_70'. The polynucleotide (e1') can be obtained from the tomato plant deposited under Accession No. FERM BP-22282 to be described below, for example.

The above description regarding the number of the "one or more" bases in the polynucleotide (e2) also applies to the number of the "one or more" bases in the polynucleotide (e2'), for example.

The above description regarding the "sequence identity" in the polynucleotide (e3) also applies to the "sequence identity" in the polynucleotide (e3'), for example.

When the polynucleotide (e) is a base sequence including SNP (e) and SNP (e') as the SNP markers, the polynucleotide (e) is, for example, the following polynucleotide (e1"), (e2"), or (e3"). The polynucleotides (e2") and (e3") are polynucleotides each having a function equivalent to that of the polynucleotide (e1") regarding the leaf mold resistance in the second resistance locus.
(e1") a polynucleotide consisting of the base sequence of SEQ ID NO: 5
(e2") a polynucleotide consisting of a base sequence obtained by deletion, substitution, insertion, and/or addition of one or more bases excluding the 40th base (A) and the 75th base (G) in the base sequence of the polynucleotide (e1")
(e3") a polynucleotide consisting of a base sequence having at least 80% sequence identity to the base sequence of the polynucleotide (e1") with the 40th base (A) and the 75th base (G) in the base sequence of the polynucleotide (e1") being conserved

In the polynucleotide (e1"), the underlined 40th base (A) in brackets and the boxed 75th base (G) in SEQ ID NO: 5 are bases corresponding to the polymorphisms of SG_70 and SG_70', respectively. The polynucleotide (e1") can be obtained from the tomato plant deposited under Accession No. FERM BP-22282 to be described below, for example.

The above description regarding the number of the "one or more" bases in the polynucleotide (e2) also applies to the number of the "one or more" bases in the polynucleotide (e2"), for example.

The above description regarding the "sequence identity" in the polynucleotide (e3) also applies to the "sequence identity" in the polynucleotide (e3"), for example.

The number of the base sequences including the SNP marker(s) in the second resistance locus is not particularly limited. For example, the second resistance locus may include any one of the polynucleotides (c), (d), and (e), or two or more (i.e., two or all three) of them.

The combination of the base sequences including the SNP marker(s) is not particularly limited, and examples thereof include the following combinations.
Combinations of two base sequences
   the polynucleotide (c) and the polynucleotide (d)
   the polynucleotide (c) and the polynucleotide (e)
   the polynucleotide (d) and the polynucleotide (e)
Combination of three base sequences
   the polynucleotide (c), the polynucleotide (d), and the polynucleotide (e)

### (2-3) Identification by base sequence in region between sites of two SNP markers

The second resistance locus may be specified by the base sequence in a region between sites of two SNP markers, as shown in (2-3) above, for example. The base sequence in a region between sites of the two SNP markers is not particularly limited, and examples thereof include a base sequence in a region between sites of two SNP markers selected from the group consisting of solcap_snp_sl_25325, solcap_snp_sl_25252, solcap_snp_sl_25252', solcap_snp_sl_35221, SG_70, SG_70', and SL10401_823 on the chromosome and a base sequence in a region between sites of two SNP markers selected from the group consisting of solcap_snp_sl_25325, solcap_snp_sl_25252, solcap_snp_sl_35221, SG_70, and SL10401_823.

Solcap_snp_sl_25325 is a polymorphism at the underlined base in brackets in SEQ ID NO: 15, for example. In the tomato plant deposited under Accession No. FERM BP-22282 to be described below, solcap_snp_sl_25325 is a polymorphism such that the underlined base in brackets is G. However, solcap_snp_sl_25325 may be a polymorphism such that the underlined base in brackets is other than G, i.e., A, T, or C, for example. The base sequence of SEQ ID NO: 15 can be obtained from the tomato plant deposited under Accession No. FERM BP-22282 to be described below, for example.

SEQ ID NO: 15
5'-
TCACGAGCAGCAAGAAAGCTATTATCGCTAGTATCCAATA[G]TACGAAAG
AGAACACTCCATCCAACATGT-3'

Solcap_snp_sl_25325 can be identified on the basis of known information in the database of the above-described web site etc., for example. The base sequence of SEQ ID NO: 16 is identical to the base sequence of Heinz except for solcap_snp_sl_25325, for example, and the underlined base in brackets is a polymorphism corresponding to solcap_snp_sl_25325. Thus, the location of solcap_snp_sl_25325 can be identified on the basis of information on the known base sequence of Heinz, for example.

SEQ ID NO: 16
5'-
TCCAATGGCATCACGAGCAGCAAGAAAGCTATTATCGCGAGTATCCAATA
[G]TACGAAAGAGAACACTCCATCCAACATGTCAACAAAATTTTCTCCATA

CT-3'

SL10401_823 is a polymorphism at the underlined base in brackets in SEQ ID NO: 17, for example. In the tomato plant deposited under Accession No. FERM BP-22282 to be described below, SL10401_823 is a polymorphism such that the underlined base in brackets is T. However, SL10401_823 may be a polymorphism such that the underlined base in brackets is other than T, i.e., A, G, or C, for example. The base sequence of SEQ ID NO: 17 can be obtained from the tomato plant deposited under Accession No. FERM BP-22282 to be described below, for example.

SEQ ID NO: 17
5'-
TACATTCTGTTTTATTTGTTCCTTACTTGTCT[T]AGTTGGGCTCATATCTC
ATTTTGAATGTAAATTACAGGTGCAAGACAA-3'

The SL10401_823 can be identified on the basis of known information in the database of the above-described web site etc., for example. The base sequence of SEQ ID NO: 18 is identical to the base sequence of Heinz except for the SL10401_823, for example, and the underlined base in brackets is a polymorphism corresponding to the SL10401_823. Thus, the location of the SL10401_823 can be identified on the basis of information on the known base sequence of Heinz, for example.

SEQ ID NO: 18
5'-
TACATTCTGTTTTATTTGTTCCTTACTTGTCT[T]AGTTGGGCTCATATCTC
ATTTTGAATGTAAATTACAGGTGCAAGACAA-3'

The upstream-side end and the downstream-side end of the region can be identified by, for example, the sites of the two SNP markers, as described above. The region is not limited as long as it extends between the sites of the two SNP markers, for example. The region may or may not include both or one of the sites of the two SNP markers, for example. When the region includes the sites of the SNP markers, the upstream-side end and the downstream-side end of the region are the sites of the SNP markers. The bases at the upstream-side end and the downstream-side end may each be the above-described underlined base in the base sequence or may be a base other than the underlined base, for example.

The two SNP markers specifying the region are not particularly limited, and examples thereof include the following combinations.
Combination of solcap_snp_sl_25325 and solcap_snp_sl_25252
Combination of solcap_snp_sl_25325 and solcap_snp_sl_25252'
Combination of solcap_snp_sl_25325 and solcap_snp_sl_35221
Combination of solcap_snp_sl_25325 and SG_70
Combination of solcap_snp_sl_25325 and SG_70'
Combination of solcap_snp_sl_25325 and SL10401_823
Combination of solcap_snp_sl_25252 and solcap_snp_sl_25252'
Combination of solcap_snp_sl_25252 and solcap_snp_sl_35221
Combination of solcap_snp_sl_25252 and SG_70
Combination of solcap_snp_sl_25252 and SG_70'
Combination of solcap_snp_sl_25252 and SL10401_823
Combination of solcap_snp_sl_25252' and solcap_snp_sl_35221
Combination of solcap_snp_sl_25252' and SG_70
Combination of solcap_snp_sl_25252' and SG_70'
Combination of solcap_snp_sl_25252' and SL10401_823
Combination of solcap_snp_sl_35221 and SG_70
Combination of solcap_snp_sl_35221 and SG_70'
Combination of solcap_snp_sl_35221 and SL10401_823
Combination of SG_70 and SG_70'
Combination of SG_70 and SL10401_823
Combination of SG_70' and SL10401_823

In the case where the second resistance locus is specified by the base sequence in a region between sites of the two SNP markers, it is preferable that the second resistance locus includes, in the base sequence in the region, the SNP marker(s) located in the region. Specifically, it is preferable that the second resistance locus includes, in the base sequence in the region, at least one SNP marker selected from the group consisting of solcap_snp_sl_25252, solcap_snp_sl_25252', solcap_snp_sl_35221, SG_70, and SG_70', for example.

The SNP marker(s) located in the region may be, for example, one or both of the sites of the two SNP markers specifying the region on the chromosome, or may be the SNP marker(s) located between the sites of the two SNP markers specifying the region. The former also is referred to as a SNP marker at the end of the region, and the latter also is referred to as a SNP marker inside the region. The SNP markers located in the region may be both the SNP markers at the ends of the region and the SNP marker inside the region, for example.

The SNP marker(s) inside the region may be, for example, a SNP marker(s) located between the SNP marker on the upstream side and the SNP marker on the downstream side specifying the region, and can be determined as appropriate on the basis of the locations of the SNP markers shown in FIG. 2, for example. The number of the SNP markers between the sites of the two SNP markers may be one or more, for example. Specifically, for example, the SNP markers may be all the SNP markers located between the sites of the SNP markers specifying the region.

The combination of the base sequence in a region between sites of the two SNP markers and the SNP marker(s) in the base sequence in the region is not particularly limited, and examples thereof include the following condition (ii).

### Condition (ii)

The second resistance locus includes a base sequence in a region between sites of solcap_snp_sl_25325 and SL10401_823 on the chromosome, and
the second resistance locus includes, in the base sequence in the region, at least one SNP marker selected from the group consisting of solcap_snp_sl_25252, solcap_snp_sl_25252', solcap_snp_sl_35221, SG_70, and SG_70'.

In the condition (ii), the number of the SNP markers inside the region is not particularly limited. For example, the region may include any one of solcap_snp_sl_25252, solcap_snp_sl_25252', solcap_snp_sl_35221, SG_70, and SG_70', or two or more (i.e., 2, 3, 4, or 5) of them. When the region includes two or more of the SNP markers, the combination of the SNP markers is not particularly limited, and examples thereof include the following combinations.
Combinations of two SNP markers:
   SNP (c) and SNP (c')
   SNP (c) and SNP (d)
   SNP (c) and SNP (e)
   SNP (c) and SNP (e')
   SNP (c') and SNP (d)
   SNP (c') and SNP (e)
   SNP (c') and SNP (e')
   SNP (d) and SNP (e)
   SNP (d) and SNP (e')
   SNP (e) and SNP (e')
Combinations of three SNP markers:
   SNP (c), SNP (c'), and SNP (d)
   SNP (c), SNP (c'), and SNP (e)
   SNP (c), SNP (c'), and SNP (e')
   SNP (c), SNP (d), and SNP (e)
   SNP (c), SNP (d), and SNP (e')
   SNP (c), SNP (e), and SNP (e')
   SNP (c'), SNP (d), and SNP (e)
   SNP (c'), SNP (d), and SNP (e')
   SNP (c'), SNP (e), and SNP (e')
   SNP (d), SNP (e), and SNP (e')
Combinations of four SNP markers:
   SNP (c), SNP (c'), SNP (d), and SNP (e)
   SNP (c), SNP (c'), SNP (d), and SNP (e')
   SNP (c), SNP (c'), SNP (e), and SNP (e')
   SNP (c), SNP (d), SNP (e), and SNP (e')
   SNP (c'), SNP (d), SNP (e), and SNP (e')
Combination of five SNP markers:
   SNP (c), SNP (c'), SNP (d), SNP (e), and SNP (e')

The resistance marker disclosed herein can confer leaf mold resistance to tomato plants, for example. In the present disclosure, the degree of the leaf mold resistance of a tomato plant can be expressed by the disease index with reference to the method described in the following Reference Information 4, for example. Regarding the calculation of the disease index according to this method, explanation in Example 1 to be described below can be referred to, and the disease index of 1 or less can be evaluated as being resistant to leaf mold and the disease index of 2 or more can be evaluated as being susceptible to leaf mold, for example. Reference Information 4: Nanshinshi, Byogaichu-dojou-hiryou-bu (Department of Pests Soils and Fertilizers in Nanshin Agricultural Experiment Station), "Ecoshot is effective for prevention and extermination of leaf mold of tomato plants", [online], Nagano Prefecture, [retrieved on March 3, 2015], the Internet <URL:
http://www.pref.nagano.lg.jp/nogi/sangyo/nogyo/gijutsu/fukyugijutsu/200802/2 00802fukyu.html>

The resistance marker disclosed herein may further include a marker for any other resistance, for example. Examples of the other resistance include root-knot nematode resistance and powdery mildew resistance.

### 2. Leaf mold resistant tomato plant

The leaf mold resistant tomato plant disclosed herein is, as described above, a leaf mold resistant tomato plant including: at least one of a leaf mold resistance locus on chromosome 1 and a leaf mold resistance locus on chromosome 6, wherein the leaf mold resistance locus on chromosome 1 is identified by at least one SNP marker selected from the group consisting of solcap_snp_sl_60160, solcap_snp_sl_8658,and solcap_snp_sl_8658', and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70'. The leaf mold resistant tomato plant disclosed herein is characterized in that: it includes at least one of a leaf mold resistance locus on chromosome 1 and a leaf mold resistance locus on chromosome 6; the leaf mold resistance locus on chromosome 1 is identified by at least one SNP marker selected from the group consisting of solcap_snp_sl_60160, solcap_snp_sl_8658, and solcap_snp_sl_8658'; and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70', and other configurations or conditions are not particularly limited. Since the leaf mold resistant tomato plant disclosed herein includes the resistance marker disclosed herein including at least one of the first resistance locus and the second resistance locus, the above description regarding the resistance marker disclosed herein also applies to the leaf mold resistant tomato plant disclosed herein, for example. In the present disclosure, the leaf mold resistance locus on chromosome 1 and the leaf mold resistance locus on chromosome 6 should be interpreted as interchangeable with the first resistance locus and the second resistance locus in the resistance marker disclosed herein, respectively, for example. The above descriptions regarding the resistance marker disclosed herein etc. also apply to the leaf mold resistant tomato plant disclosed herein, for example.

The leaf mold resistant tomato plant according to the present disclosure is resistant to leaf mold.

In the leaf mold resistant tomato plant of the present disclosure, the leaf mold resistance is conferred by at least one of the first resistance locus on chromosome 1 and the second resistance locus on chromosome 6. The leaf mold resistant tomato plant of the present disclosure includes at least one of the first resistance locus and the second resistance locus, and the first resistance locus is on chromosome 1 and the second resistance locus is on chromosome 6. However, when the leaf mold resistant tomato plant of the present disclosure includes the first resistance locus, the leaf mold resistant tomato plant of the present disclosure may include the first resistance locus on, instead of chromosome 1, any chromosome other than chromosome 1, for example. That is, the leaf mold resistant tomato plant of the present disclosure may include the first resistance locus on any of chromosome 2, chromosome 3, chromosome 4, chromosome 5, chromosome 6, chromosome 7, chromosome 8, chromosome 9, chromosome 10, chromosome 11, and chromosome 12, for example. When the leaf mold resistant tomato plant of the present disclosure includes the second resistance locus, the leaf mold resistant tomato plant of the present disclosure may include the second leaf mold resistance locus on, instead of chromosome 6, any chromosome other than chromosome 6, for example. That is, the leaf mold resistant tomato plant of the present disclosure may include the second resistance locus on any of chromosome 1, chromosome 2, chromosome 3, chromosome 4, chromosome 5, chromosome 7, chromosome 8, chromosome 9, chromosome 10, chromosome 11, and chromosome 12, for example. When the leaf mold resistant tomato plant of the present disclosure includes both the first resistance locus and the second resistance locus, the tomato plant of the present disclosure may include the first resistance locus and the second resistance locus on the same chromosome, or may include the first resistance locus and the second resistance locus on different chromosomes.

In the leaf mold resistant tomato plant according to the present disclosure, the descriptions regarding the first resistance locus and the second resistance locus provided above in connection with the resistance marker according to the present disclosure is applicable to the first resistance locus and the second resistance locus in the leaf mold resistant tomato plant of the present disclosure, for example.

The leaf mold resistant tomato plant of the present disclosure may include only the first resistance locus or only the second resistance locus. As described above, when a tomato plant including the resistance marker includes both the first resistance locus and the second resistance locus, the tomato plant exhibits further improved leaf mold resistance, for example. Specifically, a tomato plant in which each of the first resistance locus and the second resistance locus is in the heterozygous form with resistant and susceptible alleles exhibits higher leaf mold resistance than a tomato plant in which the first resistance locus is in the resistance-type homozygous form and exhibits equivalent leaf mold resistance to a tomato plant in which the second resistance locus is in the resistance-type homozygous form, for example. Also, with this configuration, for example, by crossing a tomato plant including the first resistance locus and the second resistance locus with a tomato plant having any other resistance, it is possible to obtain a tomato plant having high leaf mold resistance and the other resistance easily. Therefore, the leaf mold resistant tomato plant of the present disclosure preferably includes the leaf mold resistance locus on chromosome 1 and the leaf mold resistance locus on chromosome 6.

The leaf mold resistant tomato plant of the present disclosure may be, for example, the tomato plant deposited under Accession No. FERM BP-22282 *(S. lycopersicum)* or a progeny line thereof. The deposited tomato plant includes the first resistance locus on chromosome 1 and the second resistance locus on chromosome 6, for example. The information on the deposit is as follows.
Type of deposit: International deposit
Name of depository institution: National Institute of Technology and
Evaluation; NITE-IPOD
Address: 2-5-8-120, Kazusakamatari, Kisarazu-shi, Chiba 292-0818, Japan Accession No. FERM BP-22282
Identifying designation: Takii7
Date of acceptance: January 28, 2015

The leaf mold resistant tomato plant according to the present disclosure also can be produced by, for example, introducing at least one of the first resistance locus and the second resistance locus to a tomato plant. The method for introducing the leaf mold resistance locus (loci) to a tomato plant is not particularly limited, and a conventionally known genetic engineering procedure may be used, for example. The leaf mold resistance locus (loci) to be introduced may be the above-described leaf mold resistance locus (loci), for example.

The characteristics of the leaf mold resistant tomato plant of the present disclosure other than the leaf mold resistance, such as, for example, morphological characteristics and biological characteristics, are not particularly limited.

The leaf mold resistant tomato plant of the present disclosure may also have any other resistance. Examples of the other resistance include root-knot nematode resistance and powdery mildew resistance.

The term "plant body" as used in the present disclosure may refer to either a plant individual representing the whole plant or a part of the plant individual. The part of the plant individual may be any of organs, tissues, cells, and propagules, for example. Examples of the organs include petals, corollas, flowers, leaves, seeds, fruits, stems, and roots. The tissue is a part of the organ, for example. The part of the plant body may be one type of organ, tissue, and/or cell, or two or more types of organs, tissues, and/or cells, for example.

### 3. Method for producing leaf mold resistant tomato plant

Next, the method for producing a leaf mold resistant tomato plant disclosed herein (also referred to simply as "production method" hereinafter) will be described. The methods to be described below are merely illustrative, and the present disclosure is by no means limited to these methods. In the present disclosure, the production method also can be referred to as "growing method", for example. Also, in the present disclosure, the leaf mold resistance locus (loci) should be interpreted as interchangeable with the leaf mold resistance marker according to the present disclosure.

As described above, the method for producing a leaf mold resistant tomato plant disclosed herein includes the following steps (a) and (b):
(a) crossing the leaf mold resistant tomato plant according to the present disclosure with another tomato plant; and
(b) selecting a leaf mold resistant tomato plant from one or more tomato plants obtained in the step (a) or progeny lines thereof.

The production method disclosed herein is characterized in that the leaf mold resistant tomato plant according to the present disclosure is used as a parent, and other steps or conditions are by no means limited. The above descriptions regarding the resistance marker according to the present disclosure etc. also apply to the production method disclosed herein, for example.

In the step (a), a leaf mold resistant tomato plant used as a first parent is not limited as long as it is the leaf mold resistant tomato plant of the present disclosure. The leaf mold resistant tomato plant preferably is the above-described tomato plant deposited under Accession No. FERM BP-22282 or a progeny line thereof, for example. The leaf mold resistant tomato plant used as the first parent in the step (a) also can be obtained by the screening method disclosed herein, for example. Thus, it is possible to provide the leaf mold resistant tomato plant by, for example, selecting it from one or more tomato plants to be examined (also referred to as "candidate tomato plants") by the following step (x) prior to the step (a), for example:
(x) selecting the leaf mold resistant tomato plant of the present disclosure from one or more tomato plants to be examined.

In the step (x), the selection of the leaf mold resistant tomato plant can be referred to as selection of the tomato plant including a leaf mold resistance locus (loci). Thus, the step (x) can be carried out by the following steps (x1) and (x2), for example.
(x1) detecting the presence or absence of any of the leaf mold resistance loci on the chromosomes of each of the one or more tomato plants to be examined; and
(x2) selecting one or more tomato plants to be examined having the leaf mold resistance locus (loci) as a leaf mold resistant tomato plant.

As described above, the selection in the step (x) is selection of the tomato plant including a leaf mold resistance locus (loci), for example. Specifically, the leaf mold resistant tomato plant can be selected by carrying out detection of the leaf mold resistance loci with respect to the one or more tomato plants to be examined. As described above in connection with the resistance marker of the present disclosure, the leaf mold resistance locus can be detected by using, for example, any of the following (1-1) to (1-3) specifying the first resistance locus or any combination thereof: (1-1) the SNP marker(s); (1-2) a base sequence including the SNP marker(s); and (1-3) a base sequence in a region between sites of two SNP markers, and any of the following (2-1) to (2-3) specifying the second resistance locus or any combination thereof: (2-1) the SNP marker(s); (2-2) a base sequence including the SNP marker(s); and (2-3) a base sequence in a region between sites of two SNP markers.

The selection in the step (x) will be described with reference to the following specific example. It is to be noted, however, that the present disclosure is not limited thereto. The descriptions regarding the resistance loci provided above in connection with the resistance marker of the present disclosure also apply to the leaf mold resistance loci in the production method disclosed herein.

The selection in the step (x) is, for example, selection of a tomato plant including at least one of the leaf mold resistance locus on chromosome 1 and the leaf mold resistance locus on chromosome 6. The leaf mold resistance locus on chromosome 1 is identified by at least one SNP marker selected from the group consisting of solcap_snp_sl_60160, solcap_snp_sl_8658, and solcap_snp_sl_8658'. The leaf mold resistance locus on chromosome 6 is identified by at least one SNP marker selected from the group consisting of solcap_snp_sl_25252, solcap_snp_sl_25252', solcap_snp_sl_35221, SG_70, and SG_70.

The selection in the step (x) may be, for example, selection of a leaf mold resistant tomato plant including the leaf mold resistance locus on chromosome 1 only or selection of a leaf mold resistant tomato plant including the leaf mold resistance locus on chromosome 6 only. As described above, when a tomato plant including the resistance marker includes both the first resistance locus and the second resistance locus, the tomato plant exhibits further improved leaf mold resistance, for example. Specifically, a tomato plant in which each of the first resistance locus and the second resistance locus is in the heterozygous form with resistant and susceptible alleles exhibits higher leaf mold resistance than a tomato plant in which the first resistance locus is in the resistance-type homozygous form and exhibits equivalent leaf mold resistance to a tomato plant in which the second resistance locus is in the resistance-type homozygous form, for example. Also, with this configuration, for example, by crossing a tomato plant including the first resistance locus and the second resistance locus with a tomato plant having any other resistance, it is possible to obtain a tomato plant having high leaf mold resistance and the other resistance easily. Therefore, the selection in the step (x) preferably is selection of a leaf mold resistant tomato plant including a leaf mold resistance gene on chromosome 1 and a leaf mold resistance gene on chromosome 6.

### (1) First resistance locus

When the selection in the step (x) is selection of the first resistance locus, the first resistance locus is selected by (1-1) the SNP marker(s), as described above. Alternatively, the first resistance locus may be selected by: (1-2) a base sequence including the SNP marker(s); (1-3) a base sequence in a region between sites of two SNP markers; or any combination of (1-1) to (1-3). When the first resistance locus is selected by any combination of (1-1) to (1-3), the combination is not particularly limited, and examples thereof include the following combinations. Although the first resistance locus is selected by (1-1), the selection of the first resistance locus is not limited thereto. For example, instead of (1-1), the first resistance locus may be selected by (1-2) or (1-3), or the combination of (1-2) and (1-3).
Combination of (1-1) and (1-2)
Combination of (1-1) and (1-3)
Combination of (1-1), (1-2), and (1-3)

### (1-1) Selection by SNP marker(s)

In the first resistance locus, the SNP marker(s) to be selected is not particularly limited. To the SNP marker(s) to be selected, the description thereon in "(1-1) Identification by SNP marker(s)" provided above in connection with the resistance marker of the present disclosure also applies, for example.

As a specific example, the selection in the step (x) is, for example, selection of a tomato plant including the leaf mold resistance locus on chromosome 1 identified by solcap_snp_sl_60160 and solcap_snp_sl_8658, preferably a tomato plant including a leaf mold resistance locus on chromosome 1 identified by solcap_snp_sl_60160, solcap_snp_sl_8658, and solcap_snp_sl_8658'.

### (1-2) Selection by base sequence including SNP marker(s)

The selection in the step (x) is, for example, selection of a leaf mold resistant tomato plant including the leaf mold resistance locus on chromosome 1, and the leaf mold resistance gene on chromosome 1 is identified by at least one of the polynucleotides (a) and (b). To the polynucleotides (a) and (b), the descriptions thereon in "(1-2) Identification by base sequence including SNP marker(s)" provided above in connection with the resistance marker of the present disclosure also apply, for example.

As a specific example, the selection in the step (x) is, for example, selection of a tomato plant including a leaf mold resistance locus on chromosome 1 identified by the polynucleotides (a) and (b).

### (1-3) Selection by base sequence in region between sites of two SNP markers

The selection in the step (x) is, for example, selection of a leaf mold resistant tomato plant including the leaf mold resistance locus on chromosome 1, and the leaf mold resistance locus on chromosome 1 includes a base sequence in a region between sites of two SNP markers selected from the group consisting of solcap_snp_sl_60250, solcap_snp_sl_60160, solcap_snp_sl_8658,solcap_snp_sl_8658', and solcap_snp_sl_60089 on the chromosome. To the base sequence in a region between sites of the two SNP markers, the description thereon in "(1-3) Identification by base sequence in region between sites of two SNP markers" provided above in connection with the resistance marker of the present disclosure also applies, for example.

As a specific example, the selection in the step (x) is, for example, selection of a tomato plant that includes the leaf mold resistance locus on chromosome 1 further including, in the base sequence in the region, at least one SNP marker selected from the group consisting of solcap_snp_sl_60160, solcap_snp_sl_8658, and solcap_snp_sl_8658'.

Alternatively, the selection in the step (x) may be, for example, selection of a tomato plant including the leaf mold resistance locus on chromosome 1 satisfying the condition (i).

The chromosome to be subjected to the detection of the presence or absence of the first resistance locus preferably is chromosome 1.

### (2) Second resistance locus

When the selection is selection of the second resistance locus, the second resistance locus is selected by (2-1) the SNP marker(s), as described above. Alternatively, the second resistance locus may be selected by: (2-2) a base sequence including the SNP marker(s); (2-3) a base sequence in a region between sites of two SNP markers; or any combination of (2-1) to (2-3). When the second resistance locus is selected by any combination of (2-1) to (2-3), the combination is not particularly limited, and examples thereof include the following combinations. Although the second resistance locus is selected by (2-1), the selection of the second resistance locus is not limited thereto. For example, instead of (2-1), the second resistance locus may be selected by (2-2) or (2-3), or the combination of (2-2) and (2-3).
Combination of (2-1) and (2-2)
Combination of (2-1) and (2-3)
Combination of (2-1), (2-2), and (2-3)

### (2-1) Selection by SNP marker(s)

In the second resistance locus, the SNP marker(s) to be selected is not particularly limited. To the SNP marker(s) to be selected, the description thereon in "(2-1) Identification by SNP marker(s)" provided above in connection with the resistance marker of the present disclosure also applies, for example.

As a specific example, the selection in the step (x) is, for example, selection of a tomato plant including the second resistance locus identified by solcap_snp_sl_25252, solcap_snp_sl_35221, and SG_70, preferably a tomato plant including the second resistance locus identified by solcap_snp_sl_25252, solcap_snp_sl_25252', solcap_snp_sl_35221, SG_70, and SG_70'.

### (2-2) Selection by base sequence including SNP marker(s)

The selection in the step (x) is, for example, selection of a leaf mold resistant tomato plant including the leaf mold resistance locus on chromosome 6, and the leaf mold resistance locus on chromosome 6 is identified by at least one selected from the group consisting of the polynucleotides (c), (d) and (e). To the polynucleotides (c), (d), and (e), the descriptions thereon in " (2-2) Identification by base sequence including SNP marker" provided above in connection with the resistance marker of the present disclosure also apply, for example.

As a specific example, the selection in the step (x) is, for example, selection of a tomato plant including the leaf mold resistance locus on chromosome 6 identified by the polynucleotides (c), (d), and (e).

### (2-3) Selection by base sequence in region between sites of two SNP markers

The selection in the step (x) is, for example, selection of a leaf mold resistant tomato plant including the leaf mold resistance locus on chromosome 6, and the leaf mold resistance locus on chromosome 6 includes a base sequence in a region between sites of two SNP markers selected from the group consisting of solcap_snp_sl_25325, solcap_snp_sl_25252, solcap_snp_sl_25252', solcap_snp_sl_35221, SG_70, SG_70', and SL10401_823 on the chromosome. To the base sequence in a region between sites of the two SNP markers, the description thereon in "(2-3) Identification by base sequence in region between sites of two SNP markers" provided above in connection with the resistance marker of the present disclosure also applies, for example.

As a specific example, the selection in the step (x) is, for example, selection of a tomato plant that includes the leaf mold resistance locus on chromosome 6 further including, in the base sequence in the region, at least one SNP marker selected from the group consisting of solcap_snp_sl_25252, solcap_snp_sl_25252', solcap_snp_sl_35221, SG_70, and SG_70'.

Alternatively, the selection in the step (x) may be, for example, selection of a tomato plant including the leaf mold resistance locus on chromosome 6 satisfying the condition (ii).

The chromosome to be subjected to the detection of the presence or absence of the second resistance locus preferably is chromosome 6.

In the step (a), a tomato plant to be used as the other parent is not particularly limited, and may be, for example, a tomato plant carrying a known leaf mold resistance gene, a tomato plant having any other resistance, or the leaf mold resistant tomato plant of the present disclosure. The tomato plant having the other resistance may be, for example, a root-knot nematode resistant tomato plant or a powdery mildew resistant tomato plant.

In the step (a), the method for crossing the leaf mold resistant tomato plant with another tomato plant is not particularly limited, and a known method can be employed.

In the step (b), tomato plants from which a leaf mold resistant tomato plant is to be selected may be the tomato plants obtained in the step (a) or progeny lines obtained from these tomato plants, for example. Specifically, for example, the tomato plants from which a leaf mold resistant tomato plant is to be selected may be the F1 tomato plants obtained by the crossing in the step (a) or their progeny lines. The progeny line may be a selfed progeny or a backcross progeny of the F1 tomato plant obtained by the crossing in the step (a), or may be a tomato plant obtained by crossing the F1 tomato plant with another tomato plant, for example.

In the step (b), the selection of a leaf mold resistant tomato plant can be achieved by, for example, examining the leaf mold resistance directly or indirectly.

In the step (b), the direct examination can be carried out by evaluating the leaf mold resistance of the obtained F1 tomato plant or a progeny line thereof on the basis of the above-described disease index, for example. Specifically, for example, the direct examination can be carried out by inoculating the F1 tomato plant or the progeny line thereof with leaf mold fungus and evaluating the leaf mold resistance on the basis of the disease index. In this case, for example, the F1 tomato plant or the progeny line showing the disease index of 1 or less can be selected as a leaf mold resistant tomato plant.

In the step (b), the selection by the indirect examination can be achieved by the following steps (b1) and (b2), for example:
(b1) detecting the presence or absence of any of the leaf mold resistance loci on the chromosomes of each of the one or more tomato plants obtained in the step (a) or progeny lines thereof; and
(b2) selecting the one or more tomato plants obtained in the step (a) or progeny lines thereof having the leaf mold resistance locus (loci) as a leaf mold resistant tomato plant(s).

The selection of the leaf mold resistant tomato plant(s) in the step (b) can be performed in the same manner as in the step (x), namely, by detecting the presence or absence of any of the leaf mold resistance loci, for example. More specifically, the selection can be performed by detecting the presence or absence of any of the leaf mold resistance loci using the molecular marker.

The production method disclosed herein preferably further includes growing the leaf mold resistant tomato plant selected in the step (b).

The tomato plant or the progeny line demonstrated to be leaf mold resistant in the above-described manner can be selected as the leaf mold resistant tomato plant.

The production method disclosed herein further may include the step of collecting seeds from the progeny line obtained from the line obtained by the crossing.

### 4. Screening method for leaf mold resistant tomato plants

As described above, the screening method for leaf mold resistant tomato plants disclosed herein (also referred to simply as "screening method" hereinafter) is a screening method including the step of: as a parent for producing a leaf mold resistant tomato plant by crossing, selecting a leaf mold resistant tomato plant including, as a leaf mold resistance marker for tomato plants, at least one of a leaf mold resistance locus on chromosome 1 and a leaf mold resistance locus on chromosome 6 from one or more tomato plants to be examined, wherein the leaf mold resistance locus on chromosome 1 is identified by at least one SNP marker selected from the group consisting of solcap_snp_sl_60160, solcap_snp_sl_8658, and solcap_snp_sl_8658', and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70'.

The screening method disclosed herein is characterized in that: it includes the step of selecting a leaf mold resistant tomato plant including, as a leaf mold resistance marker for tomato plants, at least one of a leaf mold resistance locus on chromosome 1 and a leaf mold resistance locus on chromosome 6 from one or more tomato plants to be examined; the leaf mold resistance locus on chromosome 1 is identified by at least one SNP marker selected from the group consisting of solcap_snp_sl_60160, solcap_snp_sl_8658, and solcap_snp_sl_8658'; and the leaf mold resistance locus on chromosome 6 is identified by at least one SNP marker selected from the group consisting of solcap_snp_sl_25252, solcap_snp_sl_25252', solcap_snp_sl_35221, SG_70, and SG_70', and other steps or conditions are by no means limited. According to the screening method of the present disclosure, a leaf mold resistant parent can be obtained by using the resistance marker of the present disclosure. The above description regarding the resistance marker according to the present disclosure etc. also applies to the screening method according to the present disclosure, for example.

To the selection of the parent, the description of the step (x) provided above in connection with the method for producing a leaf mold resistant tomato plant according to the present disclosure also applies, for example. Examples

The present disclosure will be described specifically below with reference to examples.

### [Example 1]

The present example analyzed the mode of inheritance of leaf mold resistance loci in novel leaf mold resistant tomato plants, identified the leaf mold resistance loci, and examined the correlation between the leaf mold resistance loci and the leaf mold resistance, in order to examine whether the leaf mold resistance loci serve as a leaf mold resistance marker for tomato plants, whether tomato plants including the resistance loci is resistant to leaf mold, and whether screening for leaf mold resistant tomato plants can be performed using the leaf mold resistance marker for tomato plants.

### (1) Deposited line

In order to develop novel tomato plants resistant to leaf mold, a large amount of seeds collected from tomato lines obtained by subculture breeding in a farm owned by TAKII & CO., LTD. were subjected to breeding, and the leaf mold resistance of the resultant tomato lines was examined. As a result, a novel leaf mold resistance tomato line (*S. lycopersicum*) exhibiting leaf mold resistance was obtained (also referred to as "produced" hereinafter). This novel leaf mold resistant tomato plant was deposited under Accession No. FERM BP-22282. Hereinafter, this leaf mold resistant tomato plant is referred to as the "deposited line".

### (2) Leaf mold resistance

A tomato plant of the deposited line (Accession No. FERM BP-22282) was crossed with a leaf mold susceptible tomato plant "Momotaro (TAKII & CO., LTD., *S. lycopersicum*)" (also referred to as "susceptible tomato plant" hereinafter), whereby an F2 segregating population made up of 114 individuals (also referred to as "114 line" hereinafter) was produced. Further, each individual of the 114 line was self-crossed to produce selfed progeny F3. A leaf mold fungus inoculation test was carried out in the following manner using 9 to 15 individuals of F3 obtained from each line. The deposited line includes solcap_snp_sl_60160, solcap_snp_sl_8658, and solcap_snp_sl_8658' on chromosome 1 in the resistance-type homozygous form and solcap_snp_sl_25252, solcap_snp_sl_25252', solcap_snp_sl_35221, SG_70, and SG_70' on chromosome 6 in the resistance-type homozygous form, as described below. The susceptible tomato plant includes solcap_snp_sl_60160, solcap_snp_sl_8658, and solcap_snp_sl_8658' on chromosome 1 in a homozygous form with susceptible alleles (referred to as "susceptible-type homozygous form" hereinafter) and solcap_snp_sl_25252, solcap_snp_sl_25252', solcap_snp_sl_35221, SG_70, and SG_70' on chromosome 6 in a susceptible-type homozygous form, as described below.

The leaf mold fungus inoculation test was carried out in the following manner with reference to the method described in the following Reference Document 5.
Reference document 5: Satoru KUROYANAGI et al., "Development of a DNA marker tightly linked to the leaf mold resistance gene, Cf-9, in tomato", Research bulletin of the Aichi Agricultural Research Center, 2010, Vol. 42, pp. 15-22.

As the leaf mold fungus, a line (leaf mold fungus line 1) provided by Wageningen University was used. First, the leaf mold fungus line 1 was inoculated into a V8 agar medium containing V8^{®} Vegetable Juice (Campbell Soup Company). After the inoculation, the leaf mold fungus line 1 was cultured at 20°C for two weeks. Next, using sterilized water, conidia of the leaf mold fungus line 1 were collected from the medium after the culture. Further, a conidial suspension was prepared by diluting the conidia with sterilized water so as to achieve a concentration of 1 × 10⁵ conidia/ml. The thus-obtained conidial suspension was used as an inoculum. In the inoculation test, tomato plants having two true leaves were used. The leaf mold fungus line 1 was inoculated into the tomato plants by spraying the conidial suspension over the tomato plants (1 ml of the conidial suspension per individual). After the inoculation, the tomato plants inoculated with the leaf mold fungus line 1 were grown at 20°C with a day length of 12 hours for 2 weeks. Then, regarding the grown tomato plants, disease investigation was carried out in the following manner. It is known that the leaf mold fungus line 1 is capable of infecting tomato plants including the following resistance genes to *Cladosporium fulvum* (Cf): Cf-2, Cf-4, Cf-5, Cf-9, and Cf-11.

The disease investigation was carried out with reference to the method described in Reference Document 4. The disease index was evaluated in accordance with the following criteria.
Disease index 0: Spore formation is not observed (highly resistant).
Disease index 1: While spore formation is observed, the area of lesions on leaves is less than 50% of the area of the leaves (resistant).
Disease index 2: Spore formation is observed, and the area of lesions on leaves is 50% or more of the area of the leaves (susceptible).

The disease index was determined for each individual of F3. Then, the disease index of the corresponding individual of the 114 line of F2 was determined according to the following equation. Disease index = [(0 × n0) + (1 × n1) + (2 × n2)]/the number of the investigated individuals In the above equation, "0, 1, and 2" each indicate a disease index, and "n₀, n₁, and n₂" indicate the number of individuals evaluated as having a disease index of 0, a disease index of 1, and a disease index of 2, respectively.

As a result, the relationship between the disease index of F2 and the frequencies of appearance of the individuals having the corresponding disease index was as follows.

| | | |
|---|---|---|
| disease index = 0 | : | 25 individuals |
| 0 < disease index ≤ 0.1 | : | 1 individual |
| 0.1 < disease index ≤ 0.5 | : | 22 individuals |
| 0.5 < disease index ≤ 1.0 | : | 32 individuals |
| 1.0 < disease index ≤ 1.5 | : | 11 individuals |
| 1.5 < disease index < 2.0 | : | 9 individuals |
| disease index = 2 | : | 14 individuals |
| Total | | 114 individuals |

From these results, it is speculated that the leaf mold resistance of the deposited line (Accession No. FERM BP-22282) is controlled by a small number of resistance loci.

### (3) Novel leaf mold resistance loci

Subsequently, DNAs were extracted from the 114 line by an ordinary method. Then, the DNAs were subjected to genotyping according to the SOLCAP SNP assay (see Reference Documents 1 to 3). Thereafter, linkage maps were prepared using software (Join Map), and linkage analysis was carried out using software (Win QTL cartographer).

Also, by web retrieval utilizing tomato genome sequences available to the public on the web site of the SOL Genomics Network (http://solgenomics.net/), the polymorphisms present uniquely in both the parents, i.e., the leaf mold resistant tomato plant (the deposited line) and the leaf mold susceptible tomato plant (Momotaro), were identified. Furthermore, in the F2 segregating population, polymorphisms seen in individuals including the polymorphisms in homozygous form and individuals including the polymorphisms in the heterozygous form were identified as SNP markers. Then, using the thus-identified SNP markers, the linkage analysis was conducted in the same manner.

As a result, on each of chromosomes 1 and 6, one region with high correlation with the disease index was identified. The region on chromosome 1 includes SNP identified by solcap_snp_sl_60160. The region on chromosome 6 includes SNP identified by SG_70. This result demonstrates that novel leaf mold resistance loci are located on chromosomes 1 and 6.

### (4) Novel leaf mold resistance loci and leaf mold resistance

From the 114 line, individuals including solcap_snp_sl_60160 on chromosome 1 in the resistance-type homozygous form, the heterozygous form, or the susceptible-type homozygous form and SG_70 on chromosome 6 in the resistance-type homozygous form, the heterozygous form, or the susceptible-type homozygous form were selected (five individuals for each). Then, the disease index was determined in the same manner as in the above item (2), except that the selected tomato plants were used instead of the 114 line and that leaf mold fungus lines 1 to 4 were used instead of the leaf mold fungus line 1. Further, the disease index was determined in the same manner, except that the deposited line or the susceptible tomato plants were used instead of the selected tomato plants. The leaf mold fungus line 2 was collected from tomato plants having developed leaf mold spontaneously in a tomato cultivation farm in Iwate Prefecture. The same inoculation test as in the above item (2) was carried out using the leaf mold fungus line 2 with respect to tomato plants including Cf-2, Cf-4, Cf-5, Cf-9, and Cf-11, respectively, and it was confirmed that the leaf mold fungus line 2 can infect the tomato plants including Cf-2 and Cf-9, respectively. Leaf mold fungus exhibiting the same infectivity as the leaf mold fungus line 2 can be obtained from the Control Station for Pests in Kumamoto Prefecture, for example. The leaf mold fungus line 3 was collected from tomato plants having developed leaf mold spontaneously in a tomato cultivation farm in Fukushima Prefecture. The same inoculation test as in the above item (2) was carried out using the leaf mold fungus line 3 with respect to tomato plants including Cf-2, Cf-4, Cf-5, Cf-9, and Cf-11, respectively, and it was confirmed that the leaf mold fungus line 3 can infect the tomato plants including Cf-4 and Cf-9, respectively. Leaf mold fungus exhibiting the same infectivity as the leaf mold fungus line 3 can be obtained from NARO Institute of Vegetable and Tea Science, for example. The leaf mold fungus line 4 was obtained from Wageningen University. It is known that the leaf mold fungus line 4 can infect tomato plants including Cf-2, Cf-4, and Cf-5, respectively.

The results obtained regarding the deposited line and the susceptible tomato plants are shown in Table 1. In Table 1, "A" means that the SNP marker is included in the resistance-type homozygous form, and "B" means that the SNP marker is included in susceptible-type homozygous form (the same applies hereinafter). As can be seen in the Table 1, the susceptible tomato plants included solcap_snp_sl_60160 on chromosome 1 in susceptible-type homozygous form and SG_70 on chromosome 6 in susceptible-type homozygous form. Also, the susceptible tomato plants exhibited a disease index of 2 with respect to all the leaf mold fungus lines and were susceptible to the leaf mold. In contrast, the deposited line included solcap_snp_sl_60160 on chromosome 1 in the resistance-type homozygous form and SG_70 on chromosome 6 in the resistance-type homozygous form. The deposited line exhibited a disease index of 0 with respect to all the leaf mold fungus lines and was highly resistant to the leaf mold. These results demonstrate that the resistance locus identified by solcap_snp_sl_60160 on chromosome 1 and the resistance locus identified by SG_70 on chromosome 6 are responsible for the leaf mold resistance. Also, from the fact that the resistance locus identified by solcap_snp_sl_60160 on chromosome 1 and the resistance locus identified by SG_70 on chromosome 6 are responsible for the leaf mold resistance, it was found that these resistance loci serve as a leaf mold resistance marker for tomato plants and that screening for leaf mold resistant tomato plants is possible by using the leaf mold resistance marker for tomato plants. Further, since the leaf mold fungus lines 1 to 4 are all capable of infecting tomato plants including known leaf mold resistance genes, it was found that the first resistance locus and the second resistance locus are effective against the leaf mold fungus lines capable of infecting tomato plants including known leaf mold resistance genes, respectively.

**[Table 1]**

| Line name | solcap_snp_ sl_60160 | SG_70 | Leaf mold fungus lines | | | |
|---|---|---|---|---|---|---|
| | | | Line 1 | Line 2 | Line 3 | Line 4 |
| Deposited line | A | A | 0 | 0 | 0 | 0 |
| Susceptible tomato plants | B | B | 2 | 2 | 2 | 2 |

Next, the results obtained when the leaf mold fungus line 1 was inoculated into tomato plants with different genotypes are shown in Table 2. In Table 2, "H" means that the SNP marker is included in the heterozygous form (the same applies hereinafter). As can be seen in Table 2, in the inoculation test using the leaf mold fungus line 1, among the tomato plants including solcap_snp_sl_60160 in the resistance-type homozygous form, the heterozygous form, or the susceptible-type homozygous form and SG_70 in the susceptible-type homozygous form, the tomato plants including solcap_snp_sl_60160 in the susceptible-type homozygous form showed many grayish white spots and partial mortality on their leaves, whereas the tomato plants including solcap_snp_sl_60160 in the resistance-type homozygous form and the heterozygous form showed only a few grayish white spots on their leaves. The tomato plants including solcap_snp_sl_60160 in the resistance-type homozygous form, the heterozygous form, and the susceptible-type homozygous form and SG_70 in the susceptible-type homozygous form exhibited disease indices of 1, 1, and 2, respectively. Among the tomato plants including solcap_snp_sl_60160 in the susceptible-type homozygous form and SG_70 in the resistance-type homozygous form, the heterozygous form, or the susceptible-type homozygous form, the tomato plants including SG_70 in the susceptible-type homozygous form showed many grayish white spots and partial mortality on their leaves, whereas the tomato plants including SG_70 in the heterozygous form showed only a few grayish white spots on their leaves, and further, the tomato plants including SG_70 in the resistance-type homozygous form showed no grayish white spots on their leaves. The tomato plants including solcap_snp_sl_60160 in the susceptible-type homozygous form and SG_70 in the resistance-type homozygous form, the heterozygous form, and the susceptible-type homozygous form exhibited disease indices of 0, 1, and 2, respectively. The tomato plants including solcap_snp_sl_60160 in the resistance-type homozygous form or the heterozygous form and SG_70 in the resistance-type homozygous form or the heterozygous form exhibited a disease index of 0 and were highly resistant to the leaf mold. Further, as can be seen in Tables 3 to 5, also when the leaf mold fungus lines 2 to 4 were inoculated into the tomato plants with the above-described different genotypes, the same results were obtained. From these results, it was found that the first resistance locus is responsible for the resistance to leaf mold and inherited dominantly. It was also found that the second resistance locus is responsible for high resistance to leaf mold and inherited partially dominantly. It was further found that, by using the first resistance locus and the second resistance locus in combination, still higher resistance to the leaf mold is obtained, so that, for example, even the tomato plants including the first resistance locus and the second resistance locus in the heterozygous forms exhibit high resistance to the leaf mold. On this account, it was also found that, by crossing a tomato plant including the first resistance locus and the second resistance locus with a tomato plant having any other resistance, it is possible to produce a tomato plant having high leaf mold resistance and the other resistance easily, for example.

**[Table 2]**

| Leaf mold fungus line 1 (race 2.4.5.9.11) | | SG_70 | | |
|---|---|---|---|---|
| | | A | H | B |
| solcap_snp_sl_60160 | A | 0 | 0 | 1 |
| | H | 0 | 0 | 1 |
| | B | 0 | 1 | 2 |

**[Table 3]**

| Leaf mold fungus line 2 (race 2.9) | | SG_70 | | |
|---|---|---|---|---|
| | | A | H | B |
| solcap_snp_sl_60160 | A | 0 | 0 | 1 |
| | H | 0 | 0 | 1 |
| | B | 0 | 1 | 2 |

**[Table 4]**

| Leaf mold fungus line 3 (race 4.9) | | SG_70 | | |
|---|---|---|---|---|
| | | A | H | B |
| solcap_snp_sl_60160 | A | 0 | 0 | 1 |
| | H | 0 | 0 | 1 |
| | B | 0 | 1 | 2 |

**[Table 5]**

| Leaf mold fungus line 4 (race 2.4.5) | | SG_70 | | |
|---|---|---|---|---|
| | | A | H | B |
| solcap_snp_sl_60160 | A | 0 | 0 | 1 |
| | H | 0 | 0 | 1 |
| | B | 0 | 1 | 2 |

### (5) Leaf mold resistance locus on chromosome 1

From the 114 line, individuals including solcap_snp_sl_60160 on chromosome 1 in the heterozygous form and SG_70 on chromosome 6 in the susceptible-type homozygous form were selected (first F2 selected line). Next, each individual of the first F2 selected line was self-crossed to produce first selfed progeny F3. Then, the thus-obtained first selfed progeny F3 were subjected to the SNP assay in the same manner as in the above item (3) to identify the bases corresponding to the polymorphisms of solcap_snp_sl_60160 and SNP markers in the vicinity of solcap_snp_sl_60160, namely, solcap_snp_sl_60303, solcap_snp_sl_60250, solcap_snp_sl_8658, solcap_snp_sl_8658', solcap_snp_sl_60089, and solcap_snp_sl_60043. With respect to these individuals, a leaf mold fungus inoculation test was carried out in the same manner as in the above item (2). Also, with respect to the deposited line and the susceptible tomato plants, the SNP assay and the inoculation test were carried out in the same manner. Among the results obtained, Table 6 shows the results obtained regarding: six individuals (plant bodies X1-1 to X1-6) that were selected from the first selfed progeny F3 and different from each other in genotype of the identified SNP markers; the deposited line; and the susceptible tomato plants. In Table 6, "A" and "H" are shaded. As can be seen in Table 6, the individuals including solcap_snp_sl_60160, solcap_snp_sl_8658, and solcap_snp_sl_8658' in the resistance-type homozygous form (A) or the heterozygous form (H) each exhibited a disease index of 1 or less. These results demonstrate that, in the first resistance locus, solcap_snp_sl_60160, solcap_snp_sl_8658, and solcap_snp_sl_8658' are SNP markers showing high correlation with the leaf mold resistance. Also, from the fact that solcap_snp_sl_60160, solcap_snp_sl_8658, and solcap_snp_sl_8658' show high correlation with the leaf mold resistance, it was found that a region between the sites of solcap_snp_sl_60250 and solcap_snp_sl_60089 (a region including the above SNP markers) on chromosome 1 shows high correlation with the leaf mold resistance. From these results, it was found that the resistance locus identified by the SNP markers solcap_snp_sl_60160, solcap_snp_sl_8658, and solcap_snp_sl_8658' and the resistance locus identified by the region between the sites of solcap_snp_sl_60250 and solcap_snp_sl_60089 serve as a leaf mold resistance marker for tomato plants, and that screening for leaf mold resistant tomato plants is possible by using the leaf mold resistance marker for tomato plants.

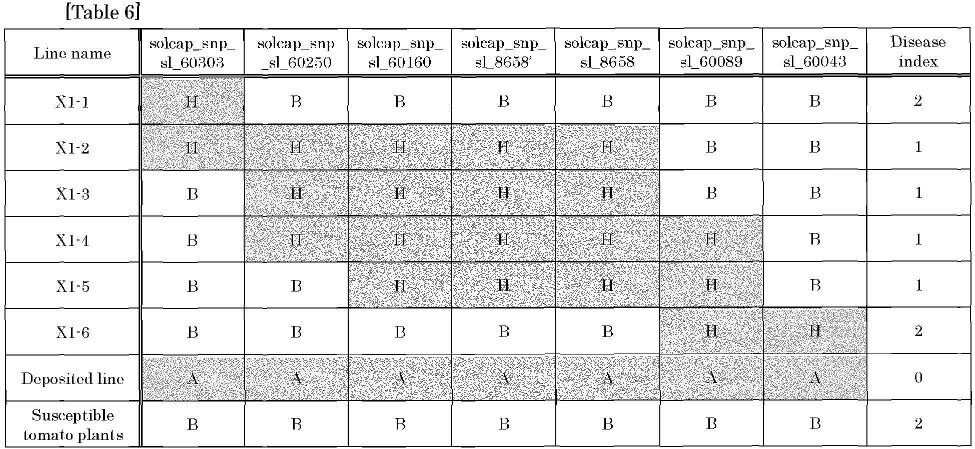

### (6) Leaf mold resistance locus on chromosome 6

From the 114 line, individuals including solcap_snp_sl_60160 on chromosome 1 in the susceptible-type homozygous form and SG_70 on chromosome 6 in the heterozygous form were selected (second F2 selected line). Next, each individual of the second F2 selected line was self-crossed to produce second selfed progeny F3. Then, the thus-obtained second selfed progeny F3 were subjected to the SNP assay in the same manner as in the above item (3) to identify the bases corresponding to the polymorphisms of SG_70 and the SNP markers in the vicinity of SG_70, namely, solcap_snp_sl_25325, solcap_snp_sl_25252, solcap_snp_sl_25252', solcap_snp_sl_35221, SG_70', and SL10401_823. With respect to these individuals, a leaf mold fungus inoculation test was carried out in the same manner as in the item (2). Also, with respect to the deposited line and the susceptible tomato plants, the SNP assay and the inoculation test were carried out in the same manner. Among the results obtained, Table 7 shows the results obtained regarding: seven individuals (plant bodies X6-1 to X6-7) that were selected from the second selfed progeny F3 and different from each other in genotype of the identified SNP markers; the deposited line; and the susceptible tomato plants. In Table 7, "A" and "H" are shaded. As can be seen in Table 7, the individuals including solcap_snp_sl_25252, solcap_snp_sl_25252', solcap_snp_sl_35221, SG_70, and SG_70' in the resistance-type homozygous form (A) or the heterozygous form (H) each exhibited a disease index of 1 or less. These results demonstrate that, in the second resistance locus, solcap_snp_sl_25252, solcap_snp_sl_25252', solcap_snp_sl_35221, SG_70, and SG_70' are SNP markers showing high correlation with the leaf mold resistance. Also, from the fact that solcap_snp_sl_25252, solcap_snp_sl_25252', solcap_snp_sl_35221, SG_70, and SG_70' show high correlation with the leaf mold resistance, it was found that a region between the sites of solcap_snp_sl_25325 and SL10401_823 (a region including the above SNP markers) on chromosome 6 shows high correlation with the leaf mold resistance. From these results, it was found that the resistance locus identified by the SNP markers solcap_snp_sl_25252, solcap_snp_sl_25252', solcap_snp_sl_35221, SG_70, and SG_70' and the resistance locus identified by the region between the sites of solcap_snp_sl_25325 and SL10401_823 serve as a leaf mold resistance marker for tomato plants, and that screening for leaf mold resistant tomato plants is possible by using the leaf mold resistance marker for tomato plants.

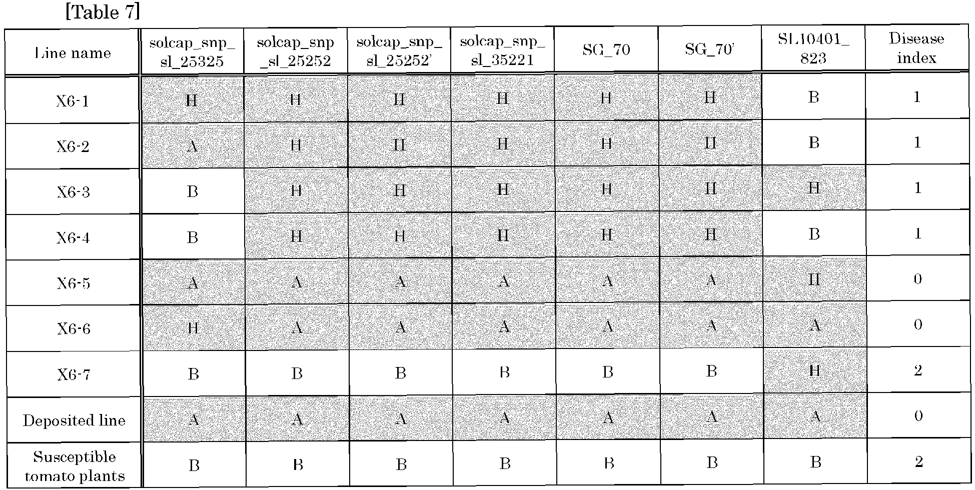

### [Example 2]

The present example examined whether novel leaf mold resistance loci are different from known leaf mold resistance genes Cf-2, Cf-4, Cf-5, Cf-9, and Cf-11.

The disease index was determined in the same manner as in the item (2) in Example 1, except that the deposited line, the susceptible tomato plants, and tomato plants including Cf genes shown in Table 8 below in the homozygous form were used instead of the 114 line and that leaf mold fungus lines 1 to 6 were used instead of the leaf mold fungus line 1. As shown in Table 8, as the leaf mold resistance gene, Vetomold includes Cf-2, Purdue 135 includes Cf-4, Ontario 7717 includes Cf-5, Ontario 7719 includes Cf-9, and Ontario 7716 includes Cf-4 and Cf-11. Also, as shown in Table 8, the respective tomato plants including the known leaf mold resistance genes were obtained from Plant Gene Resources of Canada (PGRC). The leaf mold fungus line 5 was collected from tomato plants having developed leaf mold spontaneously in a tomato cultivation farm in Gifu Prefecture. The same inoculation test as in the item (2) in Example 1 was carried out using the leaf mold fungus line 5 with respect to the tomato plants including Cf-2, Cf-4, Cf-5, Cf-9, and Cf-11, respectively, and it was confirmed that the leaf mold fungus line 5 cannot infect the tomato plants including the Cf genes, respectively. As leaf mold fungus exhibiting the same infectivity as the leaf mold fungus line 5, the leaf mold fungus registered under MAFF No. 712002 in the NARO Genebank can be used, for example. The leaf mold fungus line 6 was collected from tomato plants having developed leaf mold spontaneously in a tomato cultivation farm in Shiga Prefecture. The same inoculation test as in the item (2) in Example 1 was carried out using the leaf mold fungus line 6 with respect to tomato plants including Cf-2, Cf-4, Cf-5, Cf-9, and Cf-11, respectively, and it was confirmed that the leaf mold fungus line 6 can infect the tomato plants including Cf-2 and Cf-4, respectively. As leaf mold fungus exhibiting the same infectivity as the leaf mold fungus line 6, the leaf mold fungus registered under MAFF No. 726530 in the NARO Genebank can be used, for example.

The results obtained are shown in Table 8. As can be seen in Table 8, the susceptible tomato plants exhibited a disease index of 2 with respect to the leaf mold fungus lines 1 to 6 and were susceptible to the leaf mold. In contrast, the deposited line exhibited a disease index of 0 with respect to the leaf mold fungus lines 1 to 6 and was resistant to the leaf mold. Vetomold was resistant to the leaf mold fungus lines 3 and 5 and susceptible to the leaf mold fungus lines 1, 2, 4, and 6. Purdue 135 was resistant to the leaf mold fungus lines 2 and 5 and susceptible to the leaf mold fungus lines 1, 3, 4, and 6. Ontario 7717 was resistant to the leaf mold fungus lines 2, 3, 5 and 6 and susceptible to the leaf mold fungus lines 1 and 4. Ontario 7719 was resistant to the leaf mold fungus lines 4 to 6 and susceptible to the leaf mold fungus lines 1 to 3. Ontario 7716 was resistant to the leaf mold fungus lines 2 to 6 and susceptible to the leaf mold fungus line 1. These results demonstrate that the first resistance locus and the second resistance locus included in the deposited line are different from the leaf mold resistance genes Cf-2, Cf-4, Cf-5, Cf-9, and Cf-11.

**[Table 8]**

| Line name | Source of supply | Resistance gene | Leaf mold fungus line | | | | | |
|---|---|---|---|---|---|---|---|---|
| | | | Line 1 (race 2.4.5. 9.11.23) | Line 2 (race 2.9) | Line 3 (race 4.9) | Line 4 (race 2.4.5) | Line 5 (race 0) | Line 6 (race 2.4) |
| Deposited line | - | - | 0 | 0 | 0 | 0 | 0 | 0 |
| Susceptible tomato plants | TAKII & CO., LTD. | None | 2 | 2 | 2 | 2 | 2 | 2 |
| Vetromold | PGRC | Cf-2 | 2 | 2 | 0 | 2 | 0 | 2 |
| Purdue 135 | PGRC | Cf-4 | 2 | 0 | 2 | 2 | 0 | 2 |
| Ontario 7717 | PGRC | Cf-5 | 2 | 0 | 0 | 2 | 0 | 0 |
| Ontario 7719 | PGRC | Cf-9 | 2 | 2 | 2 | 0 | 0 | 0 |
| Ontario 7716 | PGRC | Cf-4, 11 | 2 | 0 | 0 | 0 | 0 | 0 |

### [Example 3]

The present example examined whether, by crossing a tomato plant including the first resistance locus and the second resistance locus disclosed herein with a tomato plant having any other resistance, a tomato plant having high leaf mold resistance and the other resistance can be produced easily.

LA2172 including the powdery mildew and root-knot nematode resistance gene Ol-4 (LA2172 was obtained from Tomato Genetics Resource Center [TGRC]) was crossed with Momotaro to produce F1. Further, the thus-produced F1 was crossed with Momotaro to produce a first backcross generation BC1, from which tomato plants including Ol-4 in the heterozygous form were selected using DNA markers described in Reference Document 6 shown below (selected BC1 line). The selected BC1 line was crossed with Momotaro to produce a second backcross generation BC2, from which tomato plants including Ol-4 in the heterozygous form were selected using the above-described DNA markers (selected BC2 line). The same crossing and selection were performed three more times, whereby tomato plants including Ol-4 in the heterozygous form were selected (selected BC5 line). Then, with respect to the selected BC5 line, self-crossing and seed collection were performed twice. From the thus-produced line, tomato plants including Ol-4 in the resistance-type homozygous form were selected using the DNA markers to produce a B line.
Reference Document 6: Alireza Seifi et.al., "Linked, if Not the Same, Mi-1 Homologues Confer Resistance to Tomato Powdery Mildew and Root-Knot Nematodes", MPMI, 2011, Vol. 24, No. 4, pp. 441-450

Then, the deposited line was crossed with the B line to produce F1. The disease index of the thus-produced F1 was determined in the same manner as in the item (2) in Example 1. Also, regarding the thus-produced F1, the bases corresponding to the polymorphisms of solcap_snp_sl_60160 and solcap_snp_sl_35221 were identified in the same manner as in the item (3) in Example 1. Further, regarding the thus-produced F1, a root-knot nematode inoculation test was carried out in the following manner to determine the root gall severity corresponding to the severity of the root-knot nematode, and also, a powdery mildew fungus inoculation test was carried out to determine the severity of the powdery mildew.

The root-knot nematode inoculation test was carried out in the following manner on the basis of the method described in the following Reference Document 7.
Reference Document 7: "Sentyu-gaku Jikken-hou (Experimental Techniques in Nematology) published on March 25, 2004 (The Japanese Nematological Society)".

As the root-knot nematodes, those derived from a single southern root-knot nematode egg mass collected from a tomato cultivation greenhouse in Yamanashi Prefecture were used. First, sterilized soil was placed in cultivation cups, and two individuals of root-knot nematode-free tomato seedlings were grown for 30 days in an incubator set at 30°C ± 1°C. Then, in the soil around the root of each tomato seedling grown for 30 days, three inoculation holes each having a diameter of 10 mm and a depth of 20 mm were formed with a glass rod. Thereafter, the root-knot nematodes were suspended in clarified water at a density of 200 root-knot nematodes/ml. 1 ml of the root-knot nematode suspension was aspirated with a Komagome pipette, and inoculation was achieved by supplying the suspension to these inoculation holes (1 ml/hole). The seedlings were further grown under the same conditions. After a lapse of 40 days from the inoculation, roots were collected from the grown individuals, washed with water, and the disease investigation was carried out in the following manner.

The disease investigation was carried out on the basis of the method described in the following Reference Document 8. As the disease index, the severity of root galls was evaluated in accordance with the following criteria. Root gall severity 0: There is no root gall.
Root gall severity 1: There are a few root galls.
Root gall severity 2: The presence of root galls is apparent.
Reference Document 8: G. B. Cap et al., "Inheritance of heat-stable resistance to Meloidogyne incognita in Lycopersicon peruvianum and its relationship to the Mi gene", Theoretical and Applied Genetics, Springer, 1993, volume 85, pp. 777-783

The powdery mildew fungus inoculation test was carried out on the basis of the method described in Reference Document 6.

The powdery mildew fungus was collected from tomato plants having developed powdery mildew spontaneously in a tomato cultivation greenhouse in Shiga Prefecture. A conidial suspension was prepared by diluting the collected powdery mildew fungus so as to achieve a concentration of 2.5 × 10⁴ conidia/ml. The thus-obtained conidial suspension was used as an inoculum. Thereafter, the conidial suspension was uniformly sprayed onto tomato seedlings inoculated with the root-knot nematode using a hand spray (1 ml of the conidial suspension per individual). 20 days after the inoculation, disease investigation was carried out in the following manner.

In the disease investigation, as the disease index, the severity of the powdery mildew was evaluated in accordance with the following criteria.
Severity 0: There were no symptoms.
Severity 1: Formation of spores of the powdery mildew fungus was observed slightly on the inoculated leaves.
Severity 2: Formation of spores of the powdery mildew fungus was observed a lot on the inoculated leaves.

Regarding the respective individuals inoculated with the root-knot nematode and the powdery mildew fungus, the root gall severity and the severity of the powdery mildew were investigated in accordance with the above-described criteria, and the root gall severity and the severity of the powdery mildew of each individual were determined according to the following equation. Severity = [(0 × n0) + (1 × n1) + (2 × n2)]/the number of the investigated individuals

In the case of the severity in the root-knot nematode inoculation test, in the above equation, "0, 1, and 2" each indicate the root gall severity, and "n₀, n₁, and n₂" indicate the number of individuals evaluated as having a root gall severity of 0, a root gall severity of 1, and a root gall severity of 2, respectively. In the case of the severity of the powdery mildew, in the above equation, "0, 1, and 2" each indicate the severity of the powdery mildew, and "n₀, n₁, and n₂" indicate the number of individuals evaluated as having a severity of 0, a severity of 1, and a severity of 2, respectively.

Except that the deposited line and the B line were used instead of the F1, the bases corresponding to the polymorphisms of solcap_snp_sl_60160 and solcap_snp_sl_35221 were identified and the disease index, the root gall severity, and the severity of the powdery mildew were determined in the same manner.

The results obtained are shown in Table 9. As can be seen in Table 9, F1 produced by crossing the deposited line resistant to the leaf mold with the B line resistant to the root-knot nematode and the powdery mildew included both solcap_snp_sl_60160 and solcap_snp_sl_35221 in the heterozygous form. F1 exhibited high resistance to the leaf mold, the root-knot nematode, and the powdery mildew. From these results, it was found that, by crossing a tomato plant including the first resistance locus and the second resistance locus with a tomato plant having any other resistance, a tomato plant having high leaf mold resistance and the other resistance can be produced easily.

**[Table 9]**

| Line name | solcap_snp_ sl_60160 | solcap_snp_ sl_35221 | Disease index (leaf mold) | Root gall severity (root-knot nematode) | Severity (powdery mildew) |
|---|---|---|---|---|---|
| Deposited line | A | A | 0 | 2 | 2 |
| B line | B | B | 2 | 0 | 0 |
| F1 | H | H | 0 | 0 | 0 |

This application claims priority from Japanese Patent Application No. 2015-093528 filed on April 30, 2015.

### Industrial Applicability

As specifically described above, the leaf mold resistance marker for tomato plants according to the present disclosure enables easy screening for leaf mold resistant tomato plants, for example. Also, the leaf mold resistant tomato plant according to the present disclosure includes, for example, at least one of a leaf mold resistance locus on chromosome 1 and a leaf mold resistance locus on chromosome 6, and thus can exhibit leaf mold resistance, for example. Further, since the leaf mold resistant tomato plant of the present disclosure includes, for example, at least one dominant resistance locus, it is possible to obtain progenies exhibiting leaf mold resistance inherited dominantly by crossing the leaf mold resistant tomato plant of the present disclosure with other tomato plants. Furthermore, the leaf mold resistant tomato plant according to the present disclosure is resistant to races of leaf mold fungus capable of infecting tomato plants including the leaf mold resistance genes disclosed in the prior art documents, for example. Thus, the present disclosure can eliminate the necessity of prevention and extermination using agricultural chemicals as performed conventionally, so that the problem of labor and cost for spraying the agricultural chemicals can be avoided, for example.

### [Sequence Listing]

TF14064WO_ST25.txt

## Claims

1. Use of a leaf mold resistance marker for identifying at least one of a leaf mold resistance locus from chromosome 6 of a tomato plant and a leaf mold resistance locus from chromosome 1 of a tomato plant,
which marker for identifying the leaf mold resistance locus from chromosome 6 of a tomato plant is at least one SNP marker selected from the group consisting of: (a) solcap_snp_sl_25252, (b) solcap_snp_sl_35221, and (c) SG_70, wherein:
(a) is a polynucleotide consisting of a base sequence of SEQ ID NO: 3;
(b) is a polynucleotide consisting of a base sequence of SEQ ID NO: 4; and
(c) is a polynucleotide consisting of a base sequence of SEQ ID NO: 5, and
which marker for identifying the leaf mold resistance locus from chromosome 1 of a tomato plant is at least one SNP marker selected from the group consisting of: (d) solcap_snp_sl_60160 and (e) solcap_snp_sl_8658,
wherein:
(d) is a polynucleotide consisting of a base sequence of SEQ ID NO: 1; and
(e) is a polynucleotide consisting of a base sequence of SEQ ID NO: 2.

2. The use of a leaf mold resistance marker according to claim 1, which marker for identifying the leaf mold resistance locus on chromosome 6 is a combination of (a) solcap_snp_sl_25252, (b) solcap_snp_sl_35221, and (c) SG_70.

3. The use of a leaf mold resistance marker according to claim 1, which marker for identifying the leaf mold resistance locus on chromosome 1 is a combination of (d) solcap_snp_sl_60160 and (e) solcap_snp_sl_8658.

4. The use of a leaf mold resistance marker according to claim 1 or 2, wherein the leaf mold resistance locus from chromosome 6 of a tomato plant comprises a base sequence in a region between sites of two SNP markers selected from the group consisting of (f) solcap_snp_sl_25325, (a) solcap_snp_sl_25252, (b) solcap_snp_sl_35221, (c) SG_70, and (g) SL10401_823 on the chromosome,
preferably between sites of (f) solcap_snp_sl_25325 and (g) SL10401_823 on the chromosome, wherein:
(f) is a polynucleotide consisting of a base sequence of SEQ ID NO: 15; and
(g) is a polynucleotide consisting of a base sequence of SEQ ID NO: 17.

5. The use of a leaf mold resistance marker according to claim 1 or 3, wherein the leaf mold resistance locus from chromosome 1 of a tomato plant comprises a base sequence in a region between sites of two SNP markers selected from the group consisting of (h) solcap_snp_sl_60250, (d) solcap_snp_sl_60160, (e) solcap_snp_sl_8658, and (i) solcap_snp_sl_60089 on the chromosome,
preferably between sites of (h) solcap_snp_sl_60250 and (i) solcap_snp_sl_60089 on the chromosome, wherein:
(h) is a polynucleotide consisting of a base sequence of SEQ ID NO: 12; and
(i) is a polynucleotide consisting of a base sequence of SEQ ID NO: 13.

6. A leaf mold resistant tomato plant comprising: at least one of a leaf mold resistance locus on chromosome 1 **characterized by** a leaf mold resistance marker as defined in any one of claims 1, 3 and 5 and a leaf mold resistance locus on chromosome 6 **characterized by** a leaf mold resistance marker as defined in any one of claims 1, 2 and 4, wherein the leaf mold resistance locus is present in a tomato plant identified by Accession No. FERM BP-22282.

7. The leaf mold resistant tomato plant according to claim 6, wherein the leaf mold resistant tomato plant is a plant body or a part thereof.

8. The leaf mold resistant tomato plant according to claim 6 or 7, wherein the leaf mold resistant tomato plant is a seed.

9. A screening method for a leaf mold resistant tomato plant, the screening method to select a parent for producing a leaf mold resistant tomato plant, the screening method comprising the step of, selecting a leaf mold resistant tomato plant comprising at least one of a leaf mold resistance locus on chromosome 1 and a leaf mold resistance locus on chromosome 6 from one or more tomato plants to be examined,
wherein the leaf mold resistance locus on chromosome 1 is identified using at least one SNP marker selected from the group consisting of (d) solcap_snp_sl_60160 and (e) solcap_snp_sl_8658, wherein:
(d) is a polynucleotide consisting of a base sequence of SEQ ID NO: 1; and
(e) is a polynucleotide consisting of a base sequence of SEQ ID NO: 2, and
the leaf mold resistance locus on chromosome 6 is identified using at least one SNP marker selected from the group consisting of (a) solcap_snp_sl_25252, (b) solcap_snp_sl_35221, and (c) SG_70, wherein:
(a) is a polynucleotide consisting of a base sequence of SEQ ID NO: 3;
(b) is a polynucleotide consisting of a base sequence of SEQ ID NO: 4; and
(c) is a polynucleotide consisting of a base sequence of SEQ ID NO: 5.

10. The screening method of claim 9, wherein the leaf mold resistance locus on chromosome 6 is identified using a combination of the following SNP markers: (a) solcap_snp_sl_25252, (b) solcap_snp_sl_35221, and (c) SG_70.

11. The screening method of claim 9, wherein the leaf mold resistance locus on chromosome 1 is identified using a combination of the following SNP markers: (d) solcap_snp_sl_60160 and (e) solcap_snp_sl_8658.

12. The screening method of claim 9 or 10, wherein the leaf mold resistance locus on chromosome 6 comprises a base sequence in a region between sites of two SNP markers selected from the group consisting of (f) solcap_snp_sl_25325, (a) solcap_snp_sl_25252, (b) solcap_snp_sl_35221, (c) SG_70, and (g) SL10401_823 on the chromosome,
preferably between sites of (f) solcap_snp_sl_25325 and (g) SL10401_823 on the chromosome, wherein:
(f) is a polynucleotide consisting of a base sequence of SEQ ID NO: 15; and
(g) is a polynucleotide consisting of a base sequence of SEQ ID NO: 17.

13. The screening method of claim 9 or 11, wherein the leaf mold resistance locus from chromosome 1 comprises a base sequence in a region between sites of two SNP markers selected from the group consisting of (h) solcap_snp_sl_60250, (d) solcap_snp_sl_60160, (e) solcap_snp_sl_8658, and (i) solcap_snp_sl_60089 on the chromosome,
preferably between sites of (h) solcap_snp_sl_60250 and (i) solcap_snp_sl_60089 on the chromosome, wherein:
(h) is a polynucleotide consisting of a base sequence of SEQ ID NO: 12; and
(i) is a polynucleotide consisting of a base sequence of SEQ ID NO: 13.

## Patentansprüche

1. Verwendung eines Markers für Blattfauleresistenz zum Identifizieren wenigstens eines eines Lokus für Blattfäuleresistenz auf Chromosom 6 einer Tomatenpflanze und eines Lokus für Blattfäuleresistenz auf Chromosom 1 einer Tomatenpflanze,
wobei der Marker zum Identifizieren des Lokus für Blattfäuleresistenz auf Chromosom 6 einer Tomatenpflanze wenigstens ein SNP-Marker ist, ausgewählt aus der Gruppe bestehend aus: (a) solcap_snp_sl_25252, (b) solcap_snp_sl_35221 und (c) SG_70, wobei:
(a) ein Polynukleotid ist, das aus einer Basensequenz von SEQ ID NO: 3 besteht;
(b) ein Polynukleotid ist, das aus einer Basensequenz von SEQ ID NO: 4 besteht; und
(c) ein Polynukleotid ist, das aus einer Basensequenz von SEQ ID NO: 5 besteht, und
wobei der Marker zum Identifizieren des Lokus für Blattfauleresistenz auf Chromosom 1 einer Tomatenpflanze wenigstens ein SNP-Marker ist, ausgewählt aus der Gruppe, bestehend aus: (d) solcap_snp_sl_60160 und (e) solcap_snp_sl_8658, wobei:
(d) ein Polynukleotid ist, das aus einer Basensequenz von SEQ ID NO: 1 besteht; und
(e) ein Polynukleotid ist, das aus einer Basensequenz von SEQ ID NO: 2 besteht.

2. Verwendung eines Markers für Blattfäuleresistenz nach Anspruch 1, wobei der Marker zum Identifizieren des Lokus für Blattfäuleresistenz auf Chromosom 6 eine Kombination aus (a) solcap_snp_sl_25252, (b) solcap_snp_sl_35221 und (c) SG_70 ist.

3. Verwendung eines Markers für Blattfäuleresistenz nach Anspruch 1, wobei der Marker zum Identifizieren des Lokus für Blattfäuleresistenz auf Chromosom 1 eine Kombination aus (d) solcap_snp_sl_60160 und (e) solcap_snp_sl_8658 ist.

4. Verwendung eines Markers für Blattfäuleresistenz nach Anspruch 1 oder 2, wobei der Lokus für Blattfauleresistenz von dem Chromosom 6 einer Tomatenpflanze eine Basensequenz in einer Region zwischen Stellen zweier SNP-Marker umfasst, ausgewählt aus der Gruppe, bestehend aus (f) solcap_snp_sl_25325, (a) solcap_snp_sl_25252, (b) solcap_snp_sl_35221, (c) SG_70 und (g) SL10401_823 auf dem Chromosom,
vorzugsweise zwischen Stellen (f) solcap_snp_sl_25325 und (g) SL10401_823 auf dem Chromosom, wobei:
(f) ein Polynukleotid ist, das aus einer Basensequenz von SEQ ID NO: 15 besteht; und
(g) ein Polynukleotid ist, das aus einer Basensequenz von SEQ ID NO: 17 besteht.

5. Verwendung eines Markers für Blattfäuleresistenz nach Anspruch 1 oder 3, wobei der Lokus für Blattfäuleresistenz von dem Chromosom 1 einer Tomatenpflanze eine Basensequenz in einer Region zwischen Stellen zweier SNP-Marker umfasst, ausgewählt aus der Gruppe, bestehend aus (h) solcap_snp_sl_60250, (d) solcap_snp_sl_60160, (e) solcap_snp_sl_8658 und (i) solcap_snp_sl_60089 auf dem Chromosom,
vorzugsweise zwischen den Stellen (h) solcap_snp_sl_60250 und (i) solcap_snp_sl_60089 auf dem Chromosom, wobei:
(h) ein Polynukleotid ist, das aus einer Basensequenz von SEQ ID NO: 12 besteht; und
(i) ein Polynukleotid ist, das aus einer Basensequenz von SEQ ID NO: 13 besteht.

6. Blattfäuleresistente Tomatenpflanze, umfassend: wenigstens einen eines Lokus für Blattfäuleresistenz auf Chromosom 1, **gekennzeichnet durch** einen Marker für Blattfäuleresistenz nach einem der Ansprüche 1, 3 und 5, und eines Lokus für Blattfäuleresistenz auf Chromosom 6, **gekennzeichnet durch** einen Marker für Blattfäuleresistenz nach einem der Ansprüche 1, 2 und 4, wobei der Lokus für Blattfäuleresistenz in einer Tomatenpflanze vorhanden ist, die durch die Zugangsnummer FERM BP-22282 identifiziert ist.

7. Blattfäuleresistente Tomatenpflanze nach Anspruch 6, wobei die blattfäuleresistente Tomatenpflanze ein Pflanzenkörper oder ein Teil davon ist.

8. Blattfäuleresistente Tomatenpflanze nach Anspruch 6 oder 7, wobei die blattfäuleresistente Tomatenpflanze ein Samen ist.

9. Screening-Verfahren für eine blattfäuleresistente Tomatenpflanze, wobei das Screening-Verfahren zum Auswählen eines Elternteils für das Herstellen einer blattfäuleresistenten Tomatenpflanze den Schritt des Auswählens einer blattfäuleresistenten Tomatenpflanze, die wenigstens einen eines Lokus für Blattfäuleresistenz auf Chromosom 1und Lokus für Blattfäuleresistenz auf Chromosom 6 umfasst, aus einer oder mehreren zu untersuchenden Tomatenpflanzen umfasst,
wobei der Lokus für Blattfäuleresistenz auf Chromosom 1 unter Verwendung wenigstens eines SNP-Markers identifiziert wird, ausgewählt aus der Gruppe, bestehend aus: (d) solcap_snp_sl_60160 und (e) solcap_snp_sl_8658, wobei:
(d) ein Polynukleotid ist, das aus einer Basensequenz von SEQ ID NO: 1 besteht; und
(e) ein Polynukleotid ist, das aus einer Basensequenz von SEQ ID NO: 2 besteht, und der Lokus für Blattfäuleresistenz auf Chromosom 6 unter Verwendung wenigstens eines SNP-Markers identifiziert wird, ausgewählt aus der Gruppe, bestehend aus (a) solcap_snp_sl_25252, (b) solcap_snp_sl_35221 und (c) SG_70, wobei:
(a) ein Polynukleotid ist, das aus einer Basensequenz von SEQ ID NO: 3 besteht;
(b) ein Polynukleotid ist, das aus einer Basensequenz von SEQ ID NO: 4 besteht; und
(c) ein Polynukleotid ist, das aus einer Basensequenz von SEQ ID NO: 5 besteht.

10. Screening-Verfahren nach Anspruch 9, wobei der Lokus für Blattfäuleresistenz auf Chromosom 6 unter Verwendung einer Kombination der folgenden SNP-Marker identifiziert wird: (a) solcap_snp_sl_25252, (b) solcap_snp_sl_35221 und (c) SG_70.

11. Screening-Verfahren nach Anspruch 9, wobei der der Lokus für Blattfäuleresistenz auf Chromosom 1 unter Verwendung einer Kombination der folgenden SNP-Marker identifiziert wird: (d) solcap_snp_sl_60160 und (e) solcap_snp_sl_8658.

12. Screening-Verfahren nach Anspruch 9 oder 10, wobei der Lokus für Blattfäuleresistenz auf Chromosom 6 eine Basensequenz in einer Region zwischen Stellen zweier SNP-Marker umfasst, ausgewählt aus der Gruppe, bestehend aus (f) solcap_snp_sl_25325, (a) solcap_snp_sl_25252, (b) solcap_snp_sl_35221, (c) SG_70 und (g) SL10401_823 auf dem Chromosom,
vorzugsweise zwischen Stellen (f) solcap_snp_sl_25325 und (g) SL10401_823 auf dem Chromosom, wobei:
(f) ein Polynukleotid ist, das aus einer Basensequenz von SEQ ID NO: 15 besteht; und
(g) ein Polynukleotid ist, das aus einer Basensequenz von SEQ ID NO: 17 besteht.

13. Screening-Verfahren nach Anspruch 9 oder 11, wobei der Lokus für Blattfäuleresistenz auf Chromosom 1 eine Basensequenz in einer Region zwischen Stellen zweier SNP-Marker umfasst, ausgewählt aus der Gruppe, bestehend aus (h) solcap_snp_sl_60250, (d) solcap_snp_sl_60160, (e) solcap_snp_sl_8658 und (i) solcap_snp_sl_60089 auf dem Chromosom,
vorzugsweise zwischen Stellen (h) solcap_snp_sl_60250 und (i) solcap_snp_sl_60089 auf dem Chromosom, wobei:
(h) ein Polynukleotid ist, das aus einer Basensequenz von SEQ ID NO: 12 besteht; und
(i) ein Polynukleotid ist, das aus einer Basensequenz von SEQ ID NO: 13 besteht.

## Revendications

1. Utilisation d'un marqueur de résistance à la cladosporiose permettant d'identifier un locus de résistance à la cladosporiose sur le chromosome 6 d'un plant de tomate et/ou un locus de résistance à la cladosporiose sur le chromosome 1 d'un plant de tomate,
lequel marqueur permettant d'identifier le locus de résistance à la cladosporiose sur le chromosome 6 d'un plant de tomate étant au moins un marqueur SNP choisi dans le groupe constitué de : (a) solcap_snp_sl_25252, (b) solcap_snp_sl_35221 et (c) SG_70, sachant que :
(a) représente un polynucléotide constitué d'une séquence de bases de SEQ ID N° : 3,
(b) représente un polynucléotide constitué d'une séquence de bases de SEQ ID N° : 4, et
(c) représente un polynucléotide constitué d'une séquence de bases de SEQ ID N° : 5, et
lequel marqueur permettant d'identifier le locus de résistance à la cladosporiose sur le chromosome 1 d'un plant de tomate étant au moins un marqueur SNP choisi dans le groupe constitué de : (d) solcap_snp_sl_60160 et (e) solcap_snp_sl_8658, sachant que :
(d) représente un polynucléotide constitué d'une séquence de bases de SEQ ID N° : 1, et
(e) représente un polynucléotide constitué d'une séquence de bases de SEQ ID N° : 2.

2. Utilisation d'un marqueur de résistance à la cladosporiose selon la revendication 1, lequel marqueur permettant d'identifier le locus de résistance à la cladosporiose sur le chromosome 6 est une combinaison de (a) solcap_snp_sl_25252, (b) solcap_snp_sl_35221 et (c) SG_70.

3. Utilisation d'un marqueur de résistance à la cladosporiose selon la revendication 1, lequel marqueur permettant d'identifier le locus de résistance à la cladosporiose sur le chromosome 1 est une combinaison de (d) solcap_snp_sl_60160 et (e) solcap_snp_sl_8658.

4. Utilisation d'un marqueur de résistance à la cladosporiose selon la revendication 1 ou 2, dans laquelle le locus de résistance à la cladosporiose sur le chromosome 6 d'un plant de tomate comprend une séquence de bases dans une région située entre les sites de deux marqueurs SNP choisis dans le groupe constitué de (f) solcap_snp_sl_25325, (a) solcap_snp_sl_25252, (b) solcap_snp_sl_35221, (c) SG_70 et (g) SL10401_823 sur le chromosome,
de préférence entre les sites de (f) solcap_snp_sl_25325 et (g) SL10401_823 sur le chromosome, sachant que :
(f) représente un polynucléotide constitué d'une séquence de bases de SEQ ID N° : 15 ; et
(g) représente un polynucléotide constitué d'une séquence de bases de SEQ ID N° : 17.

5. Utilisation d'un marqueur de résistance à la cladosporiose selon la revendication 1 ou 3, dans laquelle le locus de résistance à la cladosporiose sur le chromosome 1 d'un plant de tomate comprend une séquence de bases dans une région située entre les sites de deux marqueurs SNP choisis dans le groupe constitué de (h) solcap_snp_sl_60250, (d) solcap_snp_sl_60160, (e) solcap_snp_sl_8658 et (i) solcap_snp_sl_60089 sur le chromosome,
de préférence entre les sites de (h) solcap_snp_sl_60250 et (i) solcap_snp_sl_60089 sur le chromosome, sachant que :
(h) représente un polynucléotide constitué d'une séquence de bases de SEQ ID N° : 12 ; et
(i) représente un polynucléotide constitué d'une séquence de bases de SEQ ID N° : 13.

6. Plant de tomate résistant à la cladosporiose, comprenant : un locus de résistance à la cladosporiose sur le chromosome 1 **caractérisé par** un marqueur de résistance à la cladosporiose défini dans l'une quelconque des revendications 1, 3 et 5, et/ou un locus de résistance à la cladosporiose sur le chromosome 6 **caractérisé par** un marqueur de résistance à la cladosporiose défini dans l'une quelconque des revendications 1, 2 et 4, le locus de résistance à la cladosporiose étant présent dans un plant de tomate identifié par le numéro d'accès FERM BP-22282.

7. Plant de tomate résistant à la cladosporiose selon la revendication 6, ledit plant de tomate résistant à la cladosporiose étant un corps végétal ou une partie de celui-ci.

8. Plant de tomate résistant à la cladosporiose selon la revendication 6 ou 7, ledit plant de tomate résistant à la cladosporiose étant une semence.

9. Procédé de sélection d'un plant de tomate résistant à la cladosporiose, le procédé de sélection visant à sélectionner un parent pour produire un plant de tomate résistant à la cladosporiose, le procédé de sélection comprenant l'étape consistant à sélectionner un plant de tomate résistant à la cladosporiose comprenant un locus de résistance à la cladosporiose sur le chromosome 1 et/ou un locus de résistance à la cladosporiose sur le chromosome 6 parmi un ou plusieurs plants de tomate à examiner,
ledit locus de résistance à la cladosporiose sur le chromosome 1 étant identifié à l'aide d'au moins un marqueur SNP choisi dans le groupe constitué de (d) solcap_snp_sl_60160 et (e) solcap_snp_sl_8658, sachant que :
(d) représente un polynucléotide constitué d'une séquence de bases de SEQ ID N° : 1, et
(e) représente un polynucléotide constitué d'une séquence de bases de SEQ ID N° : 2, et
ledit locus de résistance à la cladosporiose sur le chromosome 6 étant identifié à l'aide d'au moins un marqueur SNP choisi dans le groupe constitué de (a) solcap_snp_sl_25252, (b) solcap_snp_sl_35221 et (c) SG_70, sachant que :
(a) représente un polynucléotide constitué d'une séquence de bases de SEQ ID N° : 3,
(b) représente un polynucléotide constitué d'une séquence de bases de SEQ ID N° : 4 ; et
(c) représente un polynucléotide constitué d'une séquence de bases de SEQ ID N° : 5.

10. Procédé de sélection selon la revendication 9, dans lequel le locus de résistance à la cladosporiose sur le chromosome 6 est identifié à l'aide d'une combinaison des marqueurs SNP suivants : (a) solcap_snp_sl_25252, (b) solcap_snp_sl_35221 et (c) SG_70.

11. Procédé de sélection selon la revendication 9, dans lequel le locus de résistance à la cladosporiose sur le chromosome 1 est identifié à l'aide d'une combinaison des marqueurs SNP suivants : (d) solcap_snp_sl_60160 et (e) solcap_snp_sl_8658.

12. Procédé de sélection selon la revendication 9 ou 10, dans lequel le locus de résistance à la cladosporiose sur le chromosome 6 comprend une séquence de bases dans une région située entre les sites de deux marqueurs SNP choisis dans le groupe constitué de (f) solcap_snp_sl_25325, (a) solcap_snp_sl_25252, (b) solcap_snp_sl_35221, (c) SG_70 et (g) SL10401_823 sur le chromosome,
de préférence entre les sites de (f) solcap_snp_sl_25325 et (g) SL10401_823 sur le chromosome, sachant que :
(f) représente un polynucléotide constitué d'une séquence de bases de SEQ ID N° : 15 ; et
(g) représente un polynucléotide constitué d'une séquence de bases de SEQ ID N° : 17.

13. Procédé de sélection selon la revendication 9 ou 11, dans lequel le locus de résistance à la cladosporiose sur le chromosome 1 comprend une séquence de bases dans une région située entre les sites de deux marqueurs SNP choisis dans le groupe constitué de (h) solcap_snp_sl_60250, (d) solcap_snp_sl_60160, (e) solcap_snp_sl_8658 et (i) solcap_snp_sl_60089 sur le chromosome,
de préférence entre les sites de (h) solcap_snp_sl_60250 et (i) solcap_snp_sl_60089 sur le chromosome, sachant que :
(h) représente un polynucléotide constitué d'une séquence de bases de SEQ ID N° : 12 ; et
(i) représente un polynucléotide constitué d'une séquence de bases de SEQ ID N° : 13.
